# EUROPEAN PATENT SPECIFICATION

(11) **EP 3 497 450 B1**
(45) Date of publication and mention of the grant of the patent: **02.07.2025**
(21) Application number: 17752361.0
(22) Date of filing: 08.08.2017
(51) Int. Cl.: G01N 33/68, G01N 33/74

(54) **HISTONES AND/OR PROADM AS MARKERS INDICATING ORGAN DYSFUNCTION**
HISTONE UND/ODER PROADM ALS MARKER ZUR ANZEIGE VON ORGANDYSFUNKTION
HISTONES ET/OU PROADM COMME MARQUEURS INDIQUANT LE DYSFONCTIONNEMENT D'ORGANE

(30) Priority: 09.08.2016 EP 16183376
(43) Date of publication of application: 19.06.2019
(73) Proprietor: B.R.A.H.M.S GmbH, 16761 Hennigsdorf (DE)
(72) Inventor: ZIERA, Tim, 14129 Berlin (DE); DREYER, Frauke, 12435 Berlin (DE); INCAMPS, Anne, 30290 Saint Victor la Coste (FR); KROP, Manne, 13509 Berlin (DE); CHARLES, Pierre-Emmanuel, 21000 Dijon (FR)
(74) Representative: Vossius & Partner Patentanwälte Rechtsanwälte mbB
(86) International application number: PCT/EP2017/070111
(87) International publication number: WO 2018/029213

(56) References cited:
- MATTHEW E. KUTCHER ET AL: "Extracellular histone release in response to traumatic injury", JOURNAL OF TRAUMA AND ACUTE CARE SURGERY, vol. 73, no. 6, 1 December 2012 (2012-12-01), pages 1389 - 1394, XP055311367, ISSN: 2163-0755, DOI: 10.1097/TA.0b013e318270d595
- MICHAEL LIEMBO EKANEY ET AL: "Impact of plasma histones in human sepsis and their contribution to cellular injury and inflammation", CRITICAL CARE, vol. 18, no. 5, 543, 24 September 2014 (2014-09-24), BIOMED CENTRAL LTD., LONDON, GB, pages 1 - 9, XP021201659, ISSN: 1364-8535, DOI: 10.1186/S13054-014-0543-8
- SIMON T. ABRAMS ET AL: "Circulating Histones Are Mediators of Trauma-associated Lung Injury", AMERICAN JOURNAL OF RESPIRATORY AND CRITICAL CARE MEDICINE., vol. 187, no. 2, 15 January 2013 (2013-01-15), US, pages 160 - 169, XP055288777, ISSN: 1073-449X, DOI: 10.1164/rccm.201206-1037OC
- KIMURA F ET AL: "Circulating Cytokines, Chemokines, and Stress Hormones are Increased in Patients with Organ Dysfunction Following Liver Resection", JOURNAL OF SURGICAL RESEARCH, ACADEMIC PRESS INC., SAN DIEGO, CA, US, vol. 133, no. 2, 15 June 2006 (2006-06-15), pages 102 - 112, XP024952706, ISSN: 0022-4804, [retrieved on 20060615], DOI: 10.1016/J.JSS.2005.10.025
- MIHAEL POTOCKI ET AL: "Mid-Regional Pro-Adrenomedullin in Acute Heart Failure: A Better Biomarker or Just Another Biomarker?", CURRENT HEART FAILURE REPORTS, CURRENT SCIENCE INC, NEW YORK, vol. 9, no. 3, 26 June 2012 (2012-06-26), CURRENT SCIENCE INC, NEW YORK, pages 244 - 251, XP035091322, ISSN: 1546-9549, DOI: 10.1007/S11897-012-0096-6
- JUN XU ET AL: "Extracellular histones are major mediators of death in sepsis", NATURE MEDICINE, vol. 15, no. 11, 1 November 2009 (2009-11-01), pages 1318 - 1321, XP055082137, ISSN: 1078-8956, DOI: 10.1038/nm.2053
- FERRUH ARTUNC ET AL: "Plasma Concentrations of the Vasoactive Peptide Fragments Mid-Regional Pro-Adrenomedullin, C-Terminal Pro-Endothelin 1 and Copeptin in Hemodialysis Patients: Associated Factors and Prediction of Mortality", PLOS ONE, vol. 9, no. 1, E86148, 22 January 2014 (2014-01-22), pages 1 - 8, XP055312685, DOI: 10.1371/journal.pone.0086148
- MEINDERS A ET AL: "P857: Preoperative serum levels of mid-regional adrenomedullin and NTproBNP predict postoperative major adverse cardiovascular events and organ failures", EUROPEAN HEART JOURNAL, vol. 33, no. Suppl. 1, 1 August 2012 (2012-08-01), OXFORD UNIVERSITY PRESS, GB, pages 130, XP009192208, ISSN: 0195-668X
- ELIZABETH D. E. PAPATHANASSOGLOU ET AL: "Association of Proinflammatory Molecules with Apoptotic Markers and Survival in Critically Ill Multiple Organ Dysfunction Patients", BIOLOGICAL RESEARCH FOR NURSING, vol. 5, no. 2, 1 October 2003 (2003-10-01), US, pages 129 - 141, XP009192196, ISSN: 1099-8004, DOI: 10.1177/1099800403257189
- MATTHEW E. KUTCHER ET AL: "Extracellular histone release in response to traumatic injury", JOURNAL OF TRAUMA AND ACUTE CARE SURGERY, vol. 73, no. 6, 1 December 2012 (2012-12-01), pages 1389 - 1394, XP055311367, ISSN: 2163-0755, DOI: 10.1097/TA.0b013e318270d595
- MICHAEL LIEMBO EKANEY ET AL: "Impact of plasma histones in human sepsis and their contribution to cellular injury and inflammation", CRITICAL CARE, vol. 18, no. 5, 543, 24 September 2014 (2014-09-24), BIOMED CENTRAL LTD., LONDON, GB, pages 1 - 9, XP021201659, ISSN: 1364-8535, DOI: 10.1186/S13054-014-0543-8
- SIMON T. ABRAMS ET AL: "Circulating Histones Are Mediators of Trauma-associated Lung Injury", AMERICAN JOURNAL OF RESPIRATORY AND CRITICAL CARE MEDICINE., vol. 187, no. 2, 15 January 2013 (2013-01-15), US, pages 160 - 169, XP055288777, ISSN: 1073-449X, DOI: 10.1164/rccm.201206-1037OC
- KIMURA F ET AL: "Circulating Cytokines, Chemokines, and Stress Hormones are Increased in Patients with Organ Dysfunction Following Liver Resection", JOURNAL OF SURGICAL RESEARCH, ACADEMIC PRESS INC., SAN DIEGO, CA, US, vol. 133, no. 2, 15 June 2006 (2006-06-15), pages 102 - 112, XP024952706, ISSN: 0022-4804, [retrieved on 20060615], DOI: 10.1016/J.JSS.2005.10.025
- MIHAEL POTOCKI ET AL: "Mid-Regional Pro-Adrenomedullin in Acute Heart Failure: A Better Biomarker or Just Another Biomarker?", CURRENT HEART FAILURE REPORTS, CURRENT SCIENCE INC, NEW YORK, vol. 9, no. 3, 26 June 2012 (2012-06-26), CURRENT SCIENCE INC, NEW YORK, pages 244 - 251, XP035091322, ISSN: 1546-9549, DOI: 10.1007/S11897-012-0096-6
- JUN XU ET AL: "Extracellular histones are major mediators of death in sepsis", NATURE MEDICINE, vol. 15, no. 11, 1 November 2009 (2009-11-01), pages 1318 - 1321, XP055082137, ISSN: 1078-8956, DOI: 10.1038/nm.2053
- FERRUH ARTUNC ET AL: "Plasma Concentrations of the Vasoactive Peptide Fragments Mid-Regional Pro-Adrenomedullin, C-Terminal Pro-Endothelin 1 and Copeptin in Hemodialysis Patients: Associated Factors and Prediction of Mortality", PLOS ONE, vol. 9, no. 1, E86148, 22 January 2014 (2014-01-22), pages 1 - 8, XP055312685, DOI: 10.1371/journal.pone.0086148
- MEINDERS A ET AL: "P857: Preoperative serum levels of mid-regional adrenomedullin and NTproBNP predict postoperative major adverse cardiovascular events and organ failures", EUROPEAN HEART JOURNAL, vol. 33, no. Suppl. 1, 1 August 2012 (2012-08-01), OXFORD UNIVERSITY PRESS, GB, pages 130, XP009192208, ISSN: 0195-668X
- ELIZABETH D. E. PAPATHANASSOGLOU ET AL: "Association of Proinflammatory Molecules with Apoptotic Markers and Survival in Critically Ill Multiple Organ Dysfunction Patients", BIOLOGICAL RESEARCH FOR NURSING, vol. 5, no. 2, 1 October 2003 (2003-10-01), US, pages 129 - 141, XP009192196, ISSN: 1099-8004, DOI: 10.1177/1099800403257189

## Description

The present invention relates to a method for the diagnosis and/or monitoring of the number of organ failures in a subject, wherein said subject is a critically ill patient. The invention relates to a method that comprises determining a level of at least one histone, particularly H2B, H4, H2A and/or H3, in a sample of said subject and wherein said level of at least one histone is indicative of said organ failure and determining a level of proadrenomedullin (proADM), particularly midregional proadrenomedullin (MR-proADM), in a sample of said subject and wherein said level of proADM is indicative of said organ failure. The invention further relates to kits for carrying out the methods of the invention.

### Background of the invention

Critically ill patients are commonly admitted to the intensive care unit (ICU) with several signs of organ dysfunction and/or organ failure (OF). In order to stratify and treat these patients quickly and effectively, clinicians need to assess the overall status and severity of illness of the patient as accurately as possible. This assessment and its resulting consequences are of high importance, since the multiple organ dysfunction syndrome (MODS) is the major cause of death on ICUs (Vincent 2006; Ferreira and Sakr 2011). Only few people die due to a single OF in the ICU (Mayr, Dunser et al. 2006). Various clinical studies support these findings and confirm a strong correlation between organ dysfunction or organ failure, morbidity and mortality of such subjects (Ferreira and Sakr 2011, Mayr et al. 2006, Vincent et al. 1998, Vincent et al. 2006).

In the 1980ies and 1990ies, many of the current gold standard ICU scoring systems were developed. Among these are outcome predictive scores, such as Acute Physiology and Chronic Health Evaluation II (APACHE II) and simplified acute physiology score (SAPSII), that are based on physiological assessment and criteria of the patient. Further scores are morbidity diagnosing scores, such as the SOFA score, that is based on organ specific parameters (Bouch and Thompson 2008). All three scores are based on at least six parameters and are determined within the first 24 hours after ICU admission. APACHE II consists of 14 variables (12 physiology parameters, age and chronic health status), each weighted from 0 to 4 with increasing abnormality of the parameter (Knaus, Draper et al. 1985). SAPS II is composed of 17 variables including 12 physiology parameters, age of the patient, type of admission and three underlying disease variables (Le Gall, Lemeshow et al. 1993). As for the SOFA score, six organs are evaluated and weighted according to their degree of dysfunction, each scoring from 0 to 4 (Vincent, Moreno et al. 1996).

Although the SOFA score was not designed to predict outcome, but rather, to display the sequential complications of ICU patients, there is a clear correlation with the mortality rate due to the cumulative organ failure in a patient (Vincent 2006). Especially, in case of a high-organ-failure group, such as patients with four or more OFs, the mortality rate was 65% or 83% in two ICU focused clinical studies compared to 6% or 22% for patients without any organ failure (Vincent, de Mendonca et al. 1998; Vincent, Sakr et al. 2006). The ability to identify subjects suffering from organ dysfunction or organ failure, and hence having a high mortality rate, is still challenging and time consuming as described in the following, but of tremendous importance. Apart from their ability to prognosticate or diagnose critically ill patients with a certain probability, the ICU scores disclosed in the prior art are used in only 10 to 15 % of non-randomly selected ICUs (often hospitals with focus on clinical studies and/or stringent quality regulations) and have several drawbacks (Breslow and Badawi 2012). For example, one disadvantage is that the scores are vulnerable to the subjectivity of every clinician performing the assessment. Hardly any hospital has either specialized scoring experts or, at least, regularly trained medical staff focusing on a reliable assessment of all parameters following strict and similar guidelines for the evaluation of the different variables of a score (Polderman, Girbes et al. 2001). Additionally, the number of parameters to assess and the resulting time to conclusion (at least one day after admission) is critical in many patients to quickly address the required treatment. Moreover, the repeated (potentially daily) score assessment is time-consuming (Le Gall, Lemeshow et al. 1993). Furthermore, the prior art scores are determined based on cumulative data with aggregated results for several parameters of a patient's status. Therefore, such score could be misinterpreted and could mislead the physician because patients with the same total score may have very distinct and divergent outcomes (Vincent, de Mendonca et al. 1998). A further unfavorable effect is the lead time bias, which reflects spontaneous variability of physiological data or effects of treatment prior to score assessment, especially when analyzing many variable parameters for calculating a total score number, which may lead to an inaccurate or biased score for outcome prediction or diagnosis (Bouch and Thompson 2008).

Kutcher et al. (2012) reports on extracellular histone release in response to traumatic injury. Ekaney et al. (2014) relates to Impact of plasma histones in human sepsis and their contribution to cellular injury and inflammation. Abrams et al. (2012) teaches that circulating histones are mediators of trauma-associated lung injury. Kimura et al. (2006) reports that circulating cytokines, chemokines, and stress hormones are increased in patients with organ dysfunction following liver resection. Potocki et al. (2012) teaches that MR-proADM is a biomarker for acute heart failure. Xu et al. (2009) reports that extracellular histones are major mediators of death in sepsis. Artunc et al. (2014) relates to the role of MR-proADM as predictor of mortality in in hemodialysis patients. Meinders et al. (2012) reports that preoperative serum levels of MR-proADM and NTproBNP predict postoperative major adverse cardiovascular events and organ failures. Papathanassoglou et al. (2003) teaches the association of proinflammatory molecules with apoptotic markers and survival in critically ill multiple organ dysfunction patients. Accordingly, there is a need of simple, fast and objective criteria for the diagnosis, prognosis, risk assessment, risk stratification, monitoring, therapy guidance and/or therapy control of organ dysfunction in a subject, particularly in a critical ill subject.

Therefore, the technical problem underlying the invention is the provision of means and methods to provide a fast and reliable way to predict organ dysfunction in a subject.

The technical problem is solved by provision of the embodiments provided herein below and as characterized in the appended claims.

### Description of the invention

The invention relates to a method for the diagnosis and/or monitoring of the number of organ failures in a subject, wherein said subject is a critically ill patient, and wherein said method comprises
(i) determining a level of at least one histone in a blood, blood plasma, or blood serum sample of said subject; and
(ii) determining a level of proadrenomedullin (proADM) in a blood, blood plasma, or blood serum sample of said subject; wherein
   (i1) said level of at least one histone is compared to a reference level of the at least one histone; and
   (ii1) said level of proADM is compared to a reference level of proADM; and
(iii) wherein the number of organ failure in said subject is identified based on the comparison in step (i1) and (ii1).Further, the invention relates to a kit for carrying out the method of the invention, wherein said kit comprises

(i) detection reagents for determining the level of at least one histone in said sample, comprising a ligand which specifically binds to said at least one histone, and
   reference data including the reference level of at least one histone, wherein an increase in the level of at least one histone in said sample as compared to the reference level of at least one histone is indicative of organ dysfunction failure in said subject; and
(ii) detection reagents for determining the level of proADM in said sample, comprising a ligand which specifically binds to said proADM, and
reference data including the reference level of proADM, wherein an increase in the level of proADM in said sample as compared to the reference level of proADM is indicative of organ failure in said subject.

The present invention solves the above identified technical problem. As documented herein below and in the appended examples, it was unexpectedly found in a clinical study that the levels of at least one histone, particularly histone H2B, H4, H2A and H3, and the level of proADM demonstrate a strong statistical relationship with organ dysfunction in the subjects; see illustrative Example 1. Accordingly, it is documented herein that said at least one histone protein and said proADM, particularly MR-proADM, can be used as a surrogate for organ dysfunction(s), particularly organ failure(s).

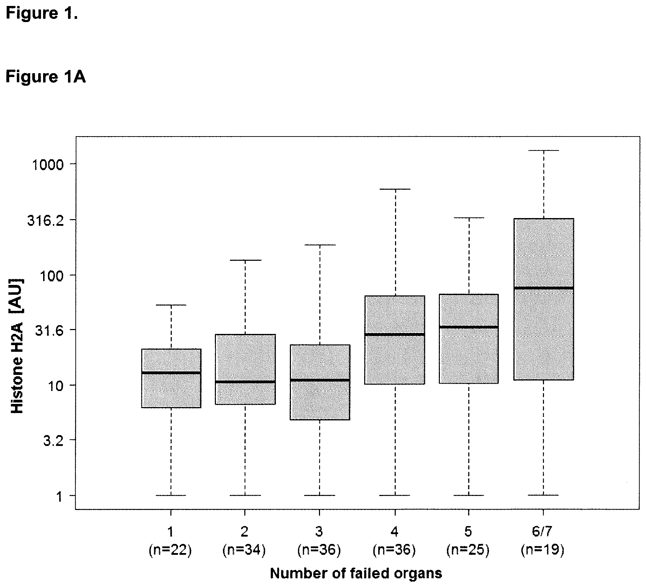

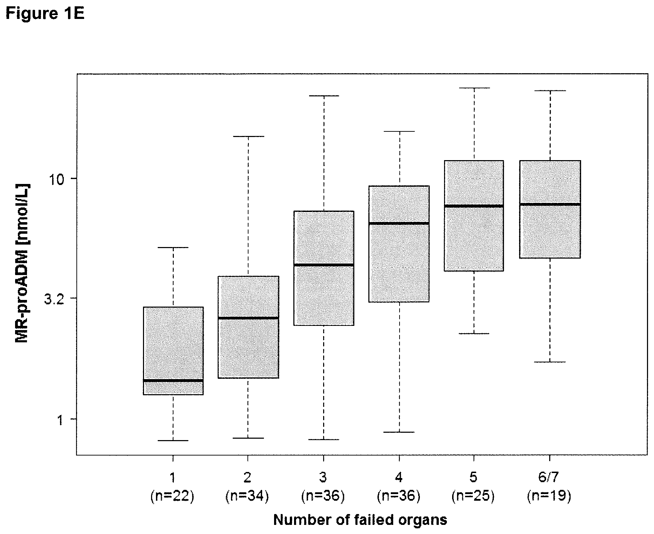

In the appended examples, it is also surprisingly demonstrated that an increase in the level of at least one histone, particularly H2B, H4, H2A and H3, in the sample of the subject indicates the organ dysfunction; see e.g. Figures 1A to 1D. In addition, it is surprisingly demonstrated that an increase in the level of the proADM, particularly of the fragment MR-proADM, in the sample of the subject indicates the organ dysfunction; see e.g. Figure 1E.

Moreover, it is documented in the appended examples that the increase of the levels of the markers increases with the number of organ dysfunctions; see e.g. Example 1. For example, it is unexpectedly shown herein below that the levels of the histones, particularly H2B, H4, H2A and H3, further increase in case four or more organ dysfunctions occur in the subject compared to the levels of subjects suffering from one to three organ dysfunctions. Accordingly, the level of the at least one histone protein is particularly indicative of four or more organ dysfunctions. Therefore, the level of the histone(s) is also particularly indicative to determine whether the subject suffers from three organ dysfunctions or four organ dysfunctions; see Table 1.

Additionally, the appended examples unexpectedly demonstrate that the level of proADM, particularly of the fragment MR-proADM, increases stepwise from one organ dysfunction, two organ dysfunctions, three organ dysfunctions to four organ dysfunctions; see e.g. Figure 1E, and Table 1. Accordingly, the level of proADM, particularly of MR-proADM, is particularly indicative of one organ dysfunction, two organ dysfunctions, three organ dysfunctions or four organ dysfunctions. Accordingly, the level of proADM, particularly of MR-proADM, is also indicative to determine whether the subject suffers from one organ dysfunction, two organ dysfunctions, three organ dysfunctions or four organ dysfunctions.

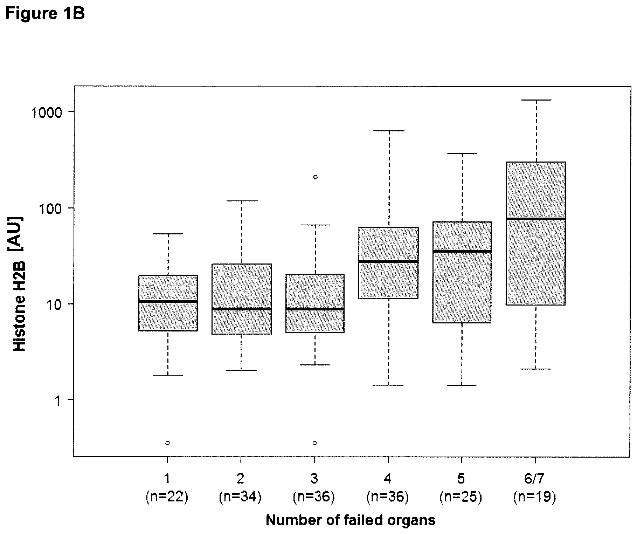

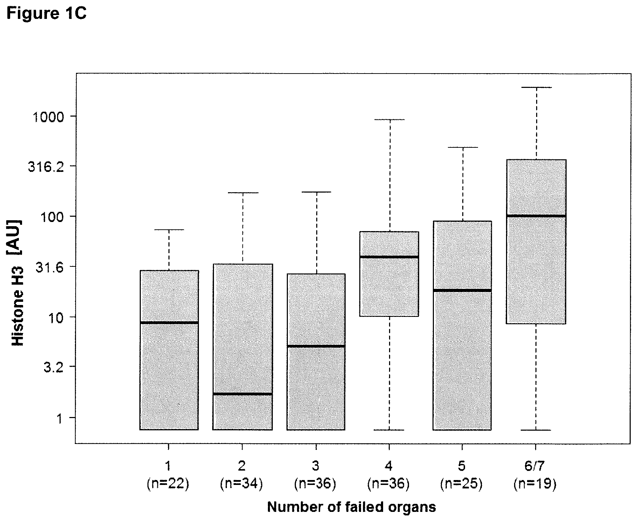

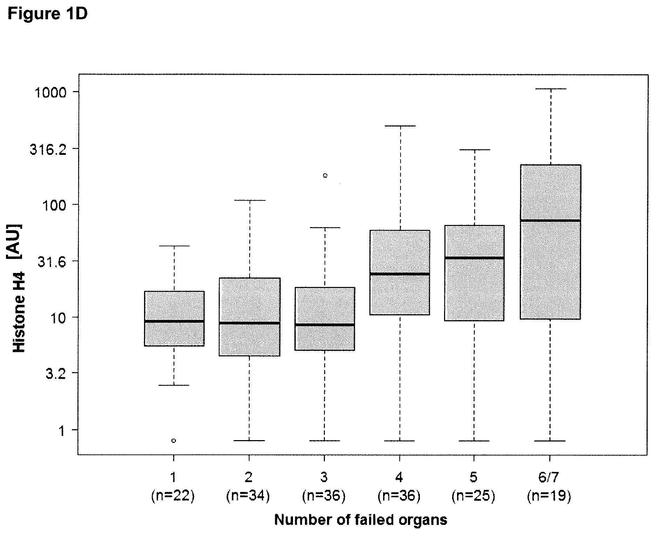

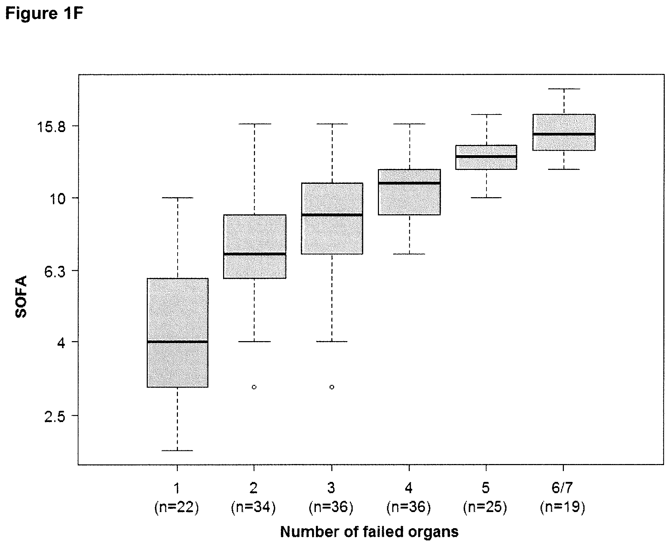

Moreover, the appended Examples demonstrate that the level of at least one histone, particularly the level of histone H2B, is indicative whether the subject suffers from one organ dysfunction, two organ dysfunctions to three organ dysfunctions or whether the subject suffers from four or more organ dysfunctions; see e.g. Figure 1 and Table 2A. In addition, it is documented herein below that the level of proADM, particularly the level of MR-proADM, is indicative whether the subject suffers from one organ dysfunction, two organ dysfunctions, three organ dysfunctions, or four or more organ dysfunctions; see e.g. Figure 1, Table 1 and Table 2A.

In addition, it is documented in the appended examples that the determination of a level of a further marker or parameter in addition to the level of at least one histone or of proADM further improves the prediction of organ dysfunction; see e.g. Table 2B. For example, the clinical scores, such as the SAPS II or the SOFA score, further improve the diagnosis. In addition, it is exemplified herein that the combination of makers further improves the diagnosis of organ dysfunction. For example, determining the level of proADM, particularly MR-proADM, and the level of at least one histone improve the prediction of organ dysfunction in the subject compared to the determination of only one marker; see illustrative Table 2B. It is further demonstrated that further markers, such as Aldolase B, procalcitonin, or lactate, improve the prediction of organ dysfunction in the subject. For example, determining the level of Aldolase B or lactate in addition to proADM increases the statistical relationship with the event of organ dysfunction in the subject. Accordingly, the invention also relates to a method comprising determining the level of a further marker and/or parameter, i.e. the use of marker panels.

The present invention has, inter alia, the following advantages over the conventional methods: the inventive methods and the kits are fast, objective, easy to use and precise for the prediction of organ failure. The methods and kits of the invention relate to markers that are easily measurable in routine in hospitals because the levels of histones and of proADM can be determined in routinely obtained blood samples or further biological fluids obtained from a subject. In addition, the determination of the levels of the histones or proADM is very fast. Therefore, the methods and the kits of the invention are suitable for a quick assessment, and diagnosis and prognosis of organ dysfunction(s). Accordingly, the quick determination also is suitable for a fast treatment decision. Furthermore, due to the simple outcome of a biomarker measurement as one specific value, there is no subjective bias of medical staff when using this method or the kits of the invention. The reproducibility is thus higher compared to subjective scoring for physiological parameters as, for example, employed in the SOFA score. The level of the histone and proADM can also be combined with further marker(s) and/or parameter(s) as add-on to existing assessments or scores in order to further improve the prediction and to adapt the analysis to specific sensitivities and specificities for evaluating the overall status of critical ill patients. When looking at patients with multiorgan failure, biomarkers may help to stratify patients according to their number of failed organs and support the diagnosis and treatment decision in this important field of ICU care.

As documented herein above and in the appended examples, the level of histones and the level of proADM were surprisingly found to correlate with organ failure in subjects. Accordingly, the present invention relates to methods and kits to determine organ failure in a subject by determining the level of proADM and (a) histone(s) in a sample of the subject.

As used herein, the term "determining the level of at least one histone" or the like refers to determining a level of a histone or a fragment thereof in a sample of the subject or determining a level of more than one histones or fragments thereof in the sample of the subject. Particularly, "determining the level of at least one histone" may refer to determining a level of a histone in the sample of the subject, wherein preferably the histone is selected from the group consisting of histone H2B, H4, H2A and H3. Particularly, the level of the histone H2B is determined. Further, "determining the level of at least one histone" may refer to determining a level of a histone in the sample of the subject, wherein particularly the level of the histone H4 is determined. Further, "determining the level of at least one histone" may refer to determining a level of two histones in the sample of the subject, wherein preferably the levels of the histones H2B and H4 are determined. Further, "determining the level of at least one histone" may refer to determining a level of three histones in the sample of the subject, wherein preferably the levels of the histones H2B, H4, and H2A are determined. Further, "determining the level of at least one histone" may refer to determining a level of four histones in the sample of the subject, wherein preferably the levels of the histones H2B, H4, H2A and H3 are determined.

Accordingly, in the context of the present invention, "determining the level of at least one histone" or the like may refer to determining a level of histone H2B, a level of histone H4, a level of histone H2A and/or a level of histone H3.

In particular, the term "determining the level of at least one histone" or the like may refer to determining a level of a histone in the sample of the subject. Accordingly, the invention also relates to a method for the diagnosis and/or monitoring of the number of organ failures in a subject, wherein said method comprises determining a level of a histone or a fragment thereof in a sample of said subject and wherein said level of a histone or said fragment thereof is indicative of said organ dysfunction.

As used herein, "histone" or "histone protein", or "histones" or "histone proteins" refers to the canonical histone(s), such as H1, H2A, H2B, H3 or H4, as well as histone variant(s), such as H3.3, H2A.Z etc. or fragment(s) thereof. Histones form the octamer around which DNA is wrapped in order to assemble the chromatin structure (Luger, Nature. 1997 Sep 18; 389(6648):251-60). For example, the histone proteins H2A, H2B, H3, and H4 (two of each) form an octamer, which is wrapped by 165 base pairs of DNA to form the fundamental subunit of chromatin, the nucleosome. Histones are also detected outside the nucleus in multiple pathophysiological processes (WO 2009/061918). The presence of extracellular histones has been described in the blood of patients suffering from different etiologies involving inflammatory processes. Histone release from activated immune cells can be mediated by extracellular traps. Activated neutrophils, as an ultimate mechanism of controlling and clearing an infection, can release extracellular fibers, so called neutrophile extracellular traps (NETs) (Brinkmann V., et al. Science 2004; 303(5663): p. 1532-5). Other mechanisms by which histones may be released into a patient's blood stream include apoptosis, necrosis, pyroptosis or necroptosis of cells.

In particular, the at least one histone is selected from the group consisting of H2B, H4, H2A and H3. Accordingly, the level of the histone to be determined in the methods and kits of the invention is particularly a level of the histones(s) H2B, H4, H2A and/or H3. The sequences of the histones are known to the skilled person. Exemplary sequences of the histones are given in SEQ ID NOs: 1 to 4. The exemplary amino acid sequence of histone H4 is given in SEQ ID NO: 1. The exemplary amino acid sequence of histone H2A is given in SEQ ID NO: 2. The exemplary amino acid sequence of histone H3 is given in SEQ ID NO: 3. The exemplary amino acid sequence of histone H2B is given in SEQ ID NO: 4. Particularly, the at least one histone is selected from the group consisting of H2B, H4, H2A and H3. More particularly, the at least one histone is selected from the group consisting of H2B, H4 and H2A. More particularly, the at least one histone is H2B and H4. More particularly, the at least one histone is H2B or H4.

It is understood that "determining the level of at least one histone" or the like refers to determining the level of at least one histone or a fragment of the at least one histone in the sample. In particular, the level of the histone H2B, H3, H2A, and/or H4 is determined in the sample. Accordingly, the at least one histone determined in the sample can be a free histone or the at least one histone determined in the sample can occur and can be assembled in a macromolecular complex, for example, in the octamer, nucleosome and/or NETs. Therefore, the level of at least one histone in the sample can comprise the level of free histone protein and/or histone protein assembled in a macromolecular complex.

A level of a histone or a fragment thereof may be determined in the sample that is not assembled in a macromolecular complex, such as a nucleosome, octamer or a neutrophil extracellular trap (NET). Such histone(s) are herein referred to as "free histone(s)". Accordingly, the level of the at least one histone may particularly be a level of at least one free histone.

The level of such free histones can be determined by the detection of amino acid sequences or structural epitopes of histones that are not accessible in an assembled stoichiometric macromolecular complex, like a mono-nucleosome or an octamer. In such structures, particular regions of the histones are covered and are thus sterically inaccessible as shown for the neutrophil extracellular traps ("NETs"), (Brinkmann V., et al. Science 303(5663): p. 1532-5, 2004). In addition, in the octamer or nucleosome, regions of histones also participate in intramolecular interactions, such as between the individual histones. Accordingly, the region/peptide/epitope of the histone that is determined may determine whether the histone is a free histone or a histone that is assembled in a macromolecular complex. For example, in an immunoassay based method, the utilized antibodies may not detect histones, e.g. H4, when they are part of the octameric core of nucleosomes as the epitopes are structurally inaccessible.

Herein below, regions/peptides/epitopes of the histone are exemplified that could be employed to determine a free histone. For example, regions/peptides/epitopes of the N-terminal or C-terminal tail of the histones can be employed to determine histones independent of whether they are assembled in the macromolecular complex or are free histones.

It is understood that the determination of histones may include post-translational modified histone proteins. Accordingly, the post-translational modifications can comprise deacetylation or acetylation, phosphorylation, methylation, ubiquitylation and citrullination of amino acids.

"Stoichiometric" in this context relates to intact complexes, e.g. a mononucleosome or an octamer. "Free histone proteins" can also comprise non-chromatin-bound histones. For example, "free histone proteins" may also comprise individual histone proteins or non-octameric histone complexes. Free histones may (e.g. transiently) be bound to individual histones, for instance, histones may form homo- or hetero-dimers. The free histones may also form homo- or hetero-tetramers. The homo- or heterotetramer may consist of four molecules of histones, e.g. H2A, H2B, H3 and/or H4. A typical heterotetramer is formed by two heterodimers, wherein each heterodimer consists of H3 and H4. It is also understood herein that a heterotetramer may be formed by H2A and H2B. It is also envisaged herein that a heterotetramer may be formed by one heterodimer consisting of H3 and H4, and one heterodimer consisting of H2A and H2B. Free histones are thus herein referred to as and can be monomeric, heterodimeric or tetrameric histone proteins, which are not assembled in a ("stoichiometric") macromolecular complex consisting of the histone octamer bound to nucleic acid, e.g. a nucleosome. In addition, free histones may also be bound to nucleic acids, and wherein said free histones are not assembled in a ("stoichiometric") macromolecular complex, e.g. an intact nucleosome. Preferably, the free histone(s) is/are essentially free of nucleic acids.

The fragment of the at least one histone can have any length, e.g. at least about 5, 10, 20, 30, 40, 50 or 100 amino acids, so long as the fragment allows the unambiguous determination of the level of the particular histone. The fragment of the at least one histone refers to an independent fragment of the histones, e.g. of the histones H2B, H4, H2A and H3. Various exemplary fragments of the histones are disclosed herein below that are suitable to determine the level of the histone in the sample of the subject. It is also herein understood that the level of the histones can be determined by determining a fragment spanning the N-terminal or C-terminal tail of the histones. In addition, the histone or the fragment thereof to be determined may also be modified, e.g. by post-translational modification. Exemplary post translational modifications can be acetylation, citrullination, deacetylation, methylation, demethylation, deimination, isomerization, phosphorylation and ubiquitination.

As used herein, the term "proadrenomedullin" or "proADM" refers to proadrenomedullin or a fragment thereof, particularly MR-proADM. It is understood that "determining the level of proADM" or the like refers to determining the level of proADM or a fragment thereof in the sample. The fragment can have any length, e.g. at least about 5, 10, 20, 30, 40, 50 or 100 amino acids, so long as the fragment allows the unambiguous determination of the level of the proADM. In particular preferred aspects of the invention, "determining the level of proADM" refers to determining the level of midregional proadrenomedullin (MR-proADM). MR-proADM is a fragment of proADM. The peptide adrenomedullin (ADM) was discovered as a hypotensive peptide comprising 52 amino acids, which had been isolated from a human phenochromocytomeby (Kitamura et al., 1993). Adrenomedullin (ADM) is encoded as a precursor peptide comprising 185 amino acids ("preproadrenomedullin" or "pre-proADM"). An exemplary amino acid sequence of ADM is given in SEQ ID NO: 5. ADM comprises the positions 95-146 of the pre-proADM amino acid sequence and is a splice product thereof. "Proadrenomedullin" ("proADM") refers to pre-proADM without the signal sequence (amino acids 1 to 21), i.e. to amino acid residues 22 to 285 of pre-proADM. "Midregional proadrenomedullin" ("MR-proADM") refers to the amino acids 42-95 of pre-proADM. An exemplary amino acid sequence of MR-proADM is given in SEQ ID NO: 6. It is also envisaged herein that a peptide and fragment thereof of pre-proADM or MR-proADM can be used for the herein described methods. For example, the peptide or the fragment thereof can comprise the amino acids 22-41 of pre-proADM (PAMP peptide) or amino acids 95-146 of pre-proADM (mature adrenomedullin). A C-terminal fragment of proADM (amino acids 153 to 185 of preproADM) is called adrenotensin. Fragments of the proADM peptides or fragments of the MR-proADM can comprise, for example, at least about 5, 10, 20, 30 or more amino acids. Accordingly, the fragment of proADM may, for example, be selected from the group consisting of MR-proADM, PAMP, adrenotensin and mature adrenomedullin, preferably herein the fragment is MR-proADM.

It is also envisaged herein that polypeptides can be determined, which have a sequence identity to proADM or to the at least one histone. For example as disclosed herein, polypeptides can be determined that have at least 75%, 80%, 85%, 90%, 91%, 92%, 93%, 94%, 95%, 96%, 97%, 98% or 99% sequence identity to SEQ ID NO: 5 or 6, or respectively to SEQ ID NO: 1, SEQ ID NO: 2, SEQ ID NO: 3, or SEQ ID NO: 4, wherein the higher values of sequence identity are preferred. As used herein, the terms "sequence identity", "homology" or "percent homology" or "identical" or "percent identity" or "percentage identity" in the context of two or more amino acid sequences may refer to two or more sequences or subsequences that are the same, or that have a specified percentage of amino acids that are the same, when compared and aligned for maximum correspondence over the window of comparison (preferably over the full length), or over a designated region as measured using a sequence comparison algorithm as known in the art, or by manual alignment and visual inspection. Sequences having, for example, 70% to 90% or greater (preferably 95% or greater) sequence identity may be considered to be substantially identical. Such a definition also applies to the complement of a test sequence. Preferably, the described identity exists over a region that is at least about 10 to about 15 amino acids in length, more preferably, over a region that is at least about 20 to about 35 amino acids in length, most preferably, over the full length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

As used herein, the "level" of the marker refers to the quantity of the molecular entity of the marker in the sample. In other words, the concentration of the marker is determined in the sample. Accordingly, the concentration of proADM or a fragment thereof, preferably MR-proADM, and the concentration of the histone(s) H2B, H4, H3 and/or H2A or (a) fragment(s) thereof is determined in the sample of the subject.

As used herein, the term "level of at least one histone" refers to the quantity of the molecular entity of the at least histone, e.g. the quantity of H2B, H4, H2A and/or H3, or a fragment thereof in a sample that is obtained from the subject. In other words, the concentration of the at least one histone protein or the fragment thereof is determined in the sample.

As used herein, the term "level of the marker proadrenomedullin (proADM)" or the "level of the marker proadrenomedullin (proADM) or a fragment thereof" refers to the quantity of the molecular entity of the marker proadrenomedullin or fragments thereof in a sample that is obtained from a subject. In other words, the concentration of the marker is determined in the sample. Hence, the term "level of the marker midregional proadrenomedullin (MR-proADM)" refers to the quantity of the molecular entity of the marker midregional proadrenomedullin (MR-proADM) in the sample that is obtained from a subject. As described above, it is also envisaged herein that a fragment of proadrenomedullin (proADM), preferably MR-proADM, can be detected and quantified. Also, fragments of MR-proADM can be detected and quantified. Suitable methods to determine the level of proADM or a fragment thereof (preferably MR-proADM) or to determine the level of the at least one histone or a fragment thereof are described herein below.

An organ is a collection of tissues joined in a structural unit to serve a common function. As used herein, the general term "organ dysfunction" can also mean that more than one organ has a dysfunction, i.e. it can also relate to organ dysfunctions unless stated otherwise. The term "organ dysfunction" or "organ dysfunctions" relates to a condition in the subject where an organ or more than one organ do(es) not perform its/their normal function compared to an unaffected organ, such for example the organ(s) of at least one healthy subject. For example, the organ(s) may have a reduced activity or the organ(s) may be abnormally active in the subject with the organ dysfunction in comparison to (an) organ(s) of at least one healthy subject. Preferably, the organ(s) with the organ dysfunction(s) may have an impaired (reduction or an increase) activity of at least about 10%, 20%, 30%, 50%, 70%, 90%, 100% or 200% compared to unaffected organ(s), e.g. of at least one healthy subject.

In particular, organ dysfunction(s) can result in organ failure(s). Accordingly, "organ dysfunction(s)" can preferably also refer to organ failure(s). "Organ failure(s)" refers to (an) organ dysfunction(s) to such a degree that normal homeostasis cannot be maintained, e.g. without external clinical intervention. "Organ failure(s)" may also refer to (an) organ dysfunction(s) to such a degree that normal homeostasis of the organ(s) cannot be maintained, e.g. without external clinical intervention. "Organ failure(s)" may also refer to (an) organ dysfunction(s) to such a degree that normal homeostasis of the subject cannot be maintained, e.g. without external clinical intervention.

As disclosed herein, the organ dysfunction may be an organ failure or at least one organ failure. The general term "organ failure" can also mean that more than one organ has a failure, i.e. it can also relate to organ failures unless stated otherwise. It is herein understood that organ dysfunctions can also be referred to as multiple organ dysfunction. It is herein understood that organ failures can also be referred to as multiple organ failure.

Exemplary organ dysfunctions or organ failures are circulatory shock, hematologic failure, liver failure, neurologic failure, renal failure, respiratory failure and metabolic acidosis. Accordingly, in the context of the invention, the organ failure or the at least one failure can preferably be selected from the group consisting of circulatory shock, hematologic failure, liver failure, neurologic failure, renal failure, and respiratory failure. It is herein understood that the subject can also have more than one organ dysfunctions or failures that are e.g. a combination of two, three, four organ dysfunctions selected from the group consisting of circulatory shock, hematologic failure, liver failure, neurologic failure, renal failure, respiratory failure and metabolic acidosis. For example, the methods and kits of the invention as defined in the claims can determine whether the subject suffers from two organ dysfunction, e.g. a circulatory shock and a respiratory failure.

As used herein, a subject with a "circulatory shock" may refer to a subject that has a systolic arterial pressure lower than about 90 mmHg with e.g. signs of peripheral hypoperfusion.

Accordingly, such a subject can have the need for infusion of vasopressor and/or inotropic agents.

As used herein, a subject with a "hematologic failure" may refer to a subject that has a thrombocythemia lower than about 100,000/mm³.

As used herein, a subject with a "liver failure" may refer to a subject that has bilirubinemia greater than about 2 mg/dL and/or enzyme levels of aspartate or alanine transaminase greater than about 500 international units per liter.

As used herein, a subject with a "neurologic failure" may refer to a subject that has Glasgow coma scale below 13.

As used herein, a subject with a "renal failure" may refer to a subject that has urine output less than about 0.5 ml/kg/h for at least about 3 hours and/or creatinemia rising more than about 50 % as compared to previous values.

As used herein, a subject with a "respiratory failure" may refer to a subject that has a ratio of the partial pressure of arterial oxygen to the fraction of inspired oxygen (Pa O₂/Fi O₂) lower than about 300 mmHg regardless of the chosen ventilatory support.

As used herein, a subject with a "metabolic acidosis" may refer to a subject with a lactate level below about 2.5 mmol/L, as base excess (base level below -2 mEquivalent/L) or bicarbonate levels (HCO₃⁻) below about 24, preferably below about 18 mmol/L.

The term "indicative of said organ dysfunction" means that the subject has or will likely have/suffer from an organ dysfunction or at least one organ dysfunction. Therefore, the level of the at least one histone and the level of proADM of the subject indicate(s) organ dysfunction in the subject.

As used herein, the term "is compared to a reference level of at least one histone" or grammatical variants thereof means that the level of the at least one histone of the subject is compared to a reference level of the at least one histone. Thus, a level of the histone of the subject is compared to a corresponding reference level of the same histone. For example, the level of the histone H2B determined in the sample of the subject is compared to a reference level of histone H2B. This applies mutatis mutandis to the other histones. The reference level of at least one histone is particularly a level of histone H2B, a level of histone H4, a level of histone H2A and/or a level of histone H3.

As used herein, the term "is compared to a reference level of proADM" or grammatical variants thereof means that the level of the proADM of the subject is compared to a reference level of the proADM. If a level of (a) fragment(s) of the at least one histone and/or of the proADM is determined the reference level may also be a level of (the) corresponding fragment(s).

As used herein, the "reference level" may reflect a normal level of the corresponding marker that is indicative of no organ dysfunction or organ failure in preferred aspects of the invention. Thus, the reference level can represent the level of the at least one histone and/or the level of proADM of a group of healthy subjects (e.g. a cohort). A healthy subject is a subject with no diagnosed (and confirmed) disease(s) and/or medical disorder(s). The healthy subjects may preferably have normally functioning organs, i.e. no organ dysfunction(s) or no organ failure(s). Accordingly, the reference level(s) can be a level of at least one histone and/or a level of proADM that is determined in samples of healthy subjects. The reference subjects or healthy subjects are herein preferably defined as a group of subjects or a group of healthy subjects, e.g. a cohort of subjects. The healthy reference subjects preferably have no organ dysfunction or no organ failure. Accordingly, the reference level is preferably a level of the at least one histone and/or a level of proADM of subjects having no organ dysfunction or no organ failure. Accordingly, the reference level is preferably a level indicating no organ dysfunction or no organ failure.

The reference level may be a level of at least one histone and/or a level of proADM of at least one reference subject, wherein said reference subject(s) has/have no organ dysfunction or no organ failure.

Further, the reference level can also be a level of at least one histone and/or a level of proADM of at least one reference subject, wherein said reference subject(s) suffer(s) from a disease and/or medical disorder, and wherein said subject(s) has/have no organ dysfunction or no organ failure.

Further, the reference level can also be a level of at least one histone and/or a level of proADM of at least one reference subject, wherein said reference subject(s) suffer(s) from a disease and/or medical disorder and an infection (such as sepsis or septic disorders), and wherein said subject(s) has/have no organ dysfunction or no organ failure.

Further, the reference level can also be a level of at least one histone and/or a level of proADM of at least one reference subject, wherein said reference subject(s) suffer(s) from a disease and/or medical disorder and not from an infection, and wherein said subject(s) has/have no organ dysfunction or no organ failure.

Further, the reference level can also be a level of at least one histone and/or a level of proADM of at least one reference subject, and wherein said reference subject(s) suffer(s) from a disease and/or medical disorder including systemic inflammatory response syndrome (SIRS), wherein said subject(s) do(es) not suffer from an infection, and wherein said subject(s) has/have no organ dysfunction or no organ failure.

As used herein, the at least one reference subject refers to more than one reference subject. Particularly, the at least one reference subject is a group or a cohort of reference subjects. As described herein below, means and methods are described to determine the levels of the markers e.g. in reference subjects and exemplary reference levels are also provided.

The reference level as used herein is typically a predetermined level, i.e. it has been determined in advance as a reference for later use at the point-of-care, e.g. ICU.

As documented herein, an increased level of at least one histone (or an increase in the level of at least one histone) and/or an increased level of proADM (or an increase in the level of proADM), particularly MR-proADM, as compared to the reference level is indicative of organ failure.

As used herein, the term "increase in the level of (the) marker" means that the level of the marker is increased, i.e. it refers to an increased level of the marker. Accordingly, the term "increase in the level of (the) marker" is used interchangeably herein with the term "increased level of (the) marker". An increased level of the marker or an increase in the level of the marker of the subject means that the level of the marker is at least about 15%, preferably at least about 20%, more preferably at least about 25%, or even more preferably at least about 30% higher than the reference level of the marker.

In the context of the invention, the term "increase in the level of at least one histone as compared to the reference level" or the like is used interchangeably with the term "increased level of the at least one histone of said subject as compared to the reference level" or the term "increased level of the at least one histone as compared to the reference level" or the like. Such terms mean that the level of the at least one histone, e.g. the level of H2B, the level of H4, the level of H2A and/or the level of H3, of the subject is at least about 15%, preferably at least about 20%, more preferably at least about 25%, or even more preferably at least about 30% higher than the reference level of the at least one histone.

As used herein, the term "increase in the level of proADM as compared to the reference level" or the like is used interchangeably herein with the term "increased level of the proADM of said subject as compared to said reference level" or the term "increased level of the proADM as compared to said reference level" or the like. Such terms mean that the level of proADM, particularly the level of MR-proADM, of the subject is at least about 15%, preferably at least about 20%, more preferably at least about 25%, or even more preferably at least about 30% higher than the reference level of proADM, particularly MR-proADM.

As used herein, "obtained from prior analysis" refers to a determination of the marker level in the sample of the same subject at a pervious time, e.g. 28 days, 7 days, 6 days, 5 days, 4 days, 3 days, 2 days or 1 day prior to the next analysis, and wherein in such aspects said previously determined level of the marker is considered as the reference level. As used herein, a further organ dysfunction may mean that a further organ shows/has a dysfunction or failure. For example, if the subject suffers from one organ dysfunction, a further organ dysfunction means that the subject suffers from two or more organ dysfunctions or failures.

The increased level of at least one histone of a subject as compared to a reference level of at least one histone can be indicative of at least one organ dysfunction, at least two organ dysfunctions, or at least three organ dysfunctions. Particularly, the increased level of at least one histone of said subject as compared to the reference level of the at least one histone can be indicative of at least five organ dysfunctions, or at least six organ dysfunctions. More particularly, the increased level of at least one histone of said subject as compared to the reference level of the at least one histone can be indicative of at least four organ dysfunctions in said subject.

The increased level of proADM of a subject as compared to a reference level of proADM can be indicative of at least one organ dysfunction. Particularly, the increased level of said proADM of said subject as compared to said reference level of proADM can be indicative of at least two organ dysfunctions. More particularly, the increased level of said proADM of said subject as compared to said reference level of proADM can be indicative of at least three organ dysfunctions. More particularly, the increased level of said proADM of said subject as compared to said reference level of proADM can be indicative of at least four organ dysfunctions.

As used herein, the term "one organ dysfunction" means that the subject has one organ dysfunction, i.e. one organ of the subject has a dysfunction. As used herein, the term "two organ dysfunctions" means that the subject has two organ dysfunctions, i.e. two organs of the subject have a dysfunction. This applies mutatis mutandis to the terms three dysfunctions, four dysfunctions, five dysfunctions or six organ dysfunctions.

Further, it is documented in the appended examples that the levels of the histones, particularly H2B, H4, H2A and H3, increase in case four or more organ dysfunctions occur in the subject compared to the levels of subjects suffering from one to three organ dysfunctions; see e.g. Figure 1 and Table 1. Therefore, the methods and kits of the invention as defined in the claims can determine how many organ dysfunctions the subject suffers from or will likely suffer from.

Accordingly, in the context of the herein provided methods and kits an increased level of said at least one histone of said subject as compared to a reference level is indicative of at least four organ dysfunctions in said subject, wherein said reference level indicates one to three organ dysfunctions.

Further, an increased level of at least one histone of a subject as compared to a reference level of the at least one histone is indicative of at least five organ dysfunctions in said subject, wherein said reference level indicates four organ dysfunctions.

Further, an increased level of at least one histone of a subject as compared to a reference level of the least one histone is indicative of at least six or seven organ dysfunctions in said subject, wherein said reference level indicates five organ dysfunctions.

Additionally, the appended examples demonstrate that the level of proADM, particularly of the fragment MR-proADM, increases stepwise from one organ dysfunction, two dysfunctions, three dysfunctions, four organ dysfunctions to five organ dysfunctions; see e.g. Figure 1E, and Table 1.

Accordingly, in the context of the herein provided methods and kits an increased level of proADM, particularly MR-proADM, of a subject as compared to a reference level of proADM is indicative of at least one organ dysfunction in said subject, wherein said reference level indicates no organ dysfunction.

Further, an increased level of proADM, particularly MR-proADM, of a subject as compared to a reference level of proADM is indicative of at least two organ dysfunctions in said subject, wherein said reference level indicates one organ dysfunction.

Further, an increased level of proADM, particularly MR-proADM, of a subject as compared to a reference level of proADM is indicative of at least three organ dysfunctions in said subject, wherein said reference level indicates two organ dysfunctions.

Further, an increased level of proADM, particularly MR-proADM, of a subject as compared to a reference level of proADM is indicative of at least four organ dysfunctions in said subject, wherein said reference level indicates three organ dysfunctions.

Further, an increased level of proADM, particularly MR-proADM, of a subject as compared to a reference level of proADM is indicative of at least five organ dysfunctions in said subject, wherein said reference level indicates four organ dysfunctions.

Further, an increased level of proADM, particularly MR-proADM, of a subject as compared to a reference level of proADM is indicative of at least six or seven organ dysfunctions in said subject, wherein said reference level indicates five organ dysfunctions.

Further, an identical or similar level, e.g. about +/-10%, +/-15%, or +/-20%, of proADM, particularly MR-proADM, and/or of at least one histone of a subject as compared to a reference level of proADM and/or the at least on histone is indicative of multiple organ dysfunctions in said subject, wherein said reference level indicates multiple organ dysfunctions.

As used herein, the term "reference level indicates number of organ dysfunction" refers to a level reflecting the number of organ dysfunctions. For example, such a (reference) level can be determined in at least one reference subject (typically in a group of subjects) that suffers from said particular number of organ dysfunctions. Such levels are documented in the appended examples, e.g. levels of markers of subjects suffering from one to six/seven organ dysfunctions. Accordingly, the reference level indicating the number of organ dysfunctions can be a level of at least one histone of at least one reference subject that suffers from the number of organ dysfunctions. Mutatis mutandis, the reference level indicating the number of dysfunctions can be a level of said proADM, particularly MR-proADM, of at least one reference subject that suffers the number of organ dysfunctions or does not suffer from an organ dysfunction.

Disclosed herein are methods and kits , wherein at least one histone is determined in the sample of the subject and wherein the level of the at least one histone is indicative whether the subject suffers from one organ dysfunction to three organ dysfunctions or whether the subject suffers from four or more organ dysfunctions. Particularly, disclosed herein are methods and kits , wherein at least one histone is determined in the sample of the subject and wherein the level of the at least one histone is indicative whether the subject suffers from one organ dysfunction or whether the subject suffers from four or more organ dysfunctions.

Particularly, disclosed herein are methods and kits, wherein at least one histone is determined in the sample of the subject and wherein the level of the at least one histone is indicative whether the subject suffers from one organ dysfunction to three organ dysfunctions or whether the subject suffers from four organ dysfunctions, or five organ dysfunctions or six and more organ dysfunctions.

In addition, it is documented herein below that the level of proADM, particularly the level of MR-proADM, is indicative whether the subject suffers from one organ dysfunction to three organ dysfunctions or whether the subject suffers from four or more organ dysfunctions; see e.g. Table 1 and Table 2A.

Accordingly, disclosed herein are methods and kits, wherein proADM, particularly MR-proADM is determined in the sample of the subject and wherein the level of proADM, particularly MR-proADM, is indicative whether the subject suffers from one organ dysfunction or whether the subject suffers two or more organ dysfunctions.

Further, disclosed herein are methods and kits, wherein proADM, particularly MR-proADM is determined in the sample of the subject and wherein the level of proADM, particularly MR-proADM, is indicative whether the subject suffers from two organ dysfunctions or whether the subject suffers from three or more organ dysfunctions.

Further, disclosed herein are methods and kits, wherein proADM, particularly MR-proADM is determined in the sample of the subject and wherein the level of proADM, particularly MR-proADM, is indicative whether the subject suffers from three organ dysfunctions or whether the subject suffers from four or more organ dysfunctions.

The reference level as used herein is typically a predetermined level, i.e. it has been determined in advance as a reference for later use at the point-of-care, e.g. ICU. It is herein understood that the reference levels and the determined marker levels (i.e. the levels that are determined for the individual subject at the point-of-care, e.g. ICU) can vary depending on the assay/method by which the levels are determined as also exemplified in table 4. For example, the reference level and the determined marker level determined by mass spectrometry based methods can be different from respective levels determined by immunoassays. The appended examples demonstrate that the levels of the markers can be determined by several methods, e.g. immunoassays and mass spectrometry based methods, e.g. table 4, and that the reference levels can additionally be optimized by statistical methods, such as the Youden's index (e.g. Rota et al., BMC Medical Research Methodology (2015) 15:24; and Ruopp et al., Biom J. 2008 June; 50(3): 419-430)). Accordingly, the skilled person is aware how to determine reference levels. For example, the levels (including reference levels) can be determined by an immunoassay, e.g. by determining the level of at least one histone and/or the level of proADM, e.g. MR-proADM, in samples of subjects (or reference subjects) that do not suffer from (an) organ dysfunctions or organ failure, or alternatively that do suffer from (an) organ dysfunctions or organ failure.

The reference levels of the at least one histone may have been determined by an immunoassay. The reference level of the at least one histone may be about 100 ng/ml, more preferably about 90 ng/ml, more preferably about 80 ng/ml, more preferably about 70 ng/ml, more preferably about 60 ng/ml, more preferably about 50 ng/ml, more preferably about 45 ng/ml, more preferably about 40 ng/ml, or most preferably about 35 ng/ml. The reference level of the at least one histone determined by an immunoassay may be about 10 ng/ml, more preferably about 15 ng/ml, more preferably about 20 ng/ml, more preferably about 25 ng/ml, more preferably about 30 ng/ml, or most preferably about 35 ng/ml.

The reference level of the at least one histone may be about 10 ng/ml to about 100 ng/ml, about 10 ng/ml to about 90 ng/ml, more preferably about 10 ng/ml to about 60 ng/ml, more preferably about 10 ng/ml to about 40 ng/ml, more preferably about 15 ng/ml to about 40 ng/ml, or most preferably about 20 ng/ml to about 40 ng/ml.

The reference level of the histone H4 determined by an immunoassay may be about 100 ng/ml, more preferably about 90 ng/ml, more preferably about 80 ng/ml, more preferably about 70 ng/ml, more preferably about 60 ng/ml, more preferably about 50 ng/ml, more preferably about 45 ng/ml, more preferably about 40 ng/ml, and most preferably about 35 ng/ml. The reference level of the histone H4 determined by an immunoassay may be about 10 ng/ml, more preferably about 15 ng/ml, more preferably about 20 ng/ml, more preferably about 25 ng/ml, more preferably about 30 ng/ml or most preferably about 35 ng/ml.

The reference level of the histone H4 may be about 10 ng/ml to about 100 ng/ml, about 10 ng/ml to about 90 ng/ml, more preferably about 10 ng/ml to about 60 ng/ml, more preferably about 10 ng/ml to about 40 ng/ml, more preferably about 15 ng/ml to about 40 ng/ml, or most more preferably about 20 ng/ml to about 40 ng/ml.

The exemplary reference levels of proADM were determined by an immunoassay as described in the appended examples. For example, the reference level of proADM may be about 4 nmol/L, more preferably about 5 nmol/L, more preferably about 7 nmol/L, more preferably about 8 nmol/L, more preferably about 9 nmol/L or particular preferably about 6 nmol/L.

An increase of the level of the marker compared to the exemplary reference level can be indicative of one organ dysfunction, two organ dysfunctions, three organ dysfunctions, four organ dysfunctions, five organ dysfunctions, or six or more organ dysfunctions in the subject.

Moreover, in another embodiment as defined in the claims, the levels (including reference levels) can be determined by mass spectrometric based methods, such as methods determining the relative quantification or determining the absolute quantification of the protein or fragment thereof of interest.

Relative quantification "rSRM" may be achieved by:
1. Determining increased or decreased presence of the target protein by comparing the SRM (selected reaction monitoring) signature peak area from a given target fragment peptide detected in the sample to the same SRM signature peak area of the target fragment peptide in at least a second, third, fourth or more biological samples.
2. Determining increased or decreased presence of target protein by comparing the SRM signature peak area from a given target peptide detected in the sample to SRM signature peak areas developed from fragment peptides from other proteins, in other samples derived from different and separate biological sources, where the SRM signature peak area comparison between the two samples for a peptide fragment are normalized for e.g to amount of protein analyzed in each sample.
3. Determining increased or decreased presence of the target protein by comparing the SRM signature peak area for a given target peptide to the SRM signature peak areas from other fragment peptides derived from different proteins within the same biological sample in order to normalize changing levels of histones protein to levels of other proteins that do not change their levels of expression under various cellular conditions.

Such assays can be applied to both unmodified fragment peptides and to modified fragment peptides of the target proteins, where the modifications include, but are not limited to phosphorylation and/or glycosylation, acetylation, methylation (mono, di, tri), citrullination, ubiquitinylation and where the relative levels of modified peptides are determined in the same manner as determining relative amounts of unmodified peptides.

Absolute quantification of a given peptide may be achieved by:
1. Comparing the SRM/MRM (multiple reaction monitoring) signature peak area for a given fragment peptide from the target proteins in an individual biological sample to the SRM/MRM signature peak area of an internal fragment peptide standard spiked into the protein lysate from the biological sample. The internal standard may be a labeled synthetic version of the fragment peptide from the target protein that is being interrogated or the labeled recombinant protein. This standard is spiked into a sample in known amounts before (mandatory for the recombinant protein) or after digestion, and the SRM/MRM signature peak area can be determined for both the internal fragment peptide standard and the native fragment peptide in the biological sample separately, followed by comparison of both peak areas. This can be applied to unmodified fragment peptides and modified fragment peptides, where the modifications include but are not limited to phosphorylation and/or glycosylation, acetylation, methylation (e.g. mono-, di-, or tri-methylation), citrullination, ubiquitinylation, and where the absolute levels of modified peptides can be determined in the same manner as determining absolute levels of unmodified peptides.
2. Peptides can also be quantified using external calibration curves. The normal curve approach uses a constant amount of a heavy peptide as an internal standard and a varying amount of light synthetic peptide spiked into the sample. A representative matrix similar to that of the test samples needs to be used to construct standard curves to account for a matrix effect. Besides, reverse curve method circumvents the issue of endogenous analyte in the matrix, where a constant amount of light peptide is spiked on top of the endogenous analyte to create an internal standard and varying amounts of heavy peptide are spiked to create a set of concentration standards. Test samples to be compared with either the normal or reverse curves are spiked with the same amount of standard peptide as the internal standard spiked into the matrix used to create the calibration curve.

Accordingly, the skilled person is aware how to determine marker levels and in particular appropriate reference levels as is also exemplified in the appended examples.

As described above, reference levels can be determined by determining the level of at least one histone and/or the level of proADM in samples of subjects suffering from e.g. one organ dysfunction, two organ dysfunctions, three organ dysfunctions, or four or more organ dysfunctions. An increased level of the target protein(s) in the sample of the subject compared to a reference level of each target protein(s) representing one or more organ dysfunctions is then indicative of the number of organ dysfunctions wherein the number of organ dysfunction in the sample of the subject is higher than the number of organ dysfunction represented by the reference levels. For example, the reference levels of at least one histone and/or of proADM being indicative of at least four organ dysfunctions are exemplified in tables 3 and 4. Accordingly, the reference level of the at least one histone being indicative of at least four organ dysfunctions may be about 100 ng/ml, more preferably about 90 ng/ml, more preferably about 80 ng/ml, more preferably about 70 ng/ml, more preferably about 60 ng/ml, more preferably about 50 ng/ml, more preferably about 45 ng/ml, more preferably about 40 ng/ml, or most preferably about 35 ng/ml. The reference level of the at least one histone being indicative of at least four organ dysfunctions may be about 10 ng/ml, more preferably about 15 ng/ml, more preferably about 20 ng/ml, more preferably about 25 ng/ml, more preferably about 30 ng/ml, or most preferably about 35 ng/ml.

The reference level of the at least one histone being indicative of at least four organ dysfunctions may be about 10 ng/ml to about 100 ng/ml, about about 10 ng/ml to about 90 ng/ml, more preferably about 10 ng/ml to about 60 ng/ml, more preferably about 10 ng/ml to about 40 ng/ml, more preferably about 15 ng/ml to about 40 ng/ml, or most more preferably about 20 ng/ml to about 40 ng/ml.

The reference level of proADM being indicative of at least four organ dysfunctions may be about 4 nmol/L, more preferably about 5 nmol/L, more preferably about 7 nmol/L, more preferably about 8 nmol/L, more preferably about 9 nmol/L or particular preferably about 6 nmol/L. An increase of the level of the marker compared to such exemplary reference levels can be indicative of at least four organ dysfunctions in the subject.

The sensitivity and specificity of the provided methods depend on more than just the analytical quality of the test. Sensitivity and specificity also depend on the definition of what constitutes an abnormal (e.g. organ dysfunction) or normal result. The specificity and sensitivity of the provided methods may also be dependent on the employed reference level as exemplified in table 4. The distribution of levels of the at least one histone and/or of proADM, preferably the level of MR-proADM, for subjects with and without an organ dysfunction may overlap. Under such conditions, a test does not absolutely distinguish normal from a dysfunctioning state with 100% accuracy. The skilled person is aware of the fact that the condition per se of a subject or at least one further maker and/or parameter of the subject can assist in the interpretation of the data and that this further information allows a more reliable prognosis in the areas of overlap. Accordingly, the level(s) of at least one further marker and/or parameter is determined. The levels of at least one further marker and/or parameter can also be compared to reference levels, wherein similar or identical values/levels of said at least one further marker and/or parameter of the subject compared to the corresponding levels of said at least one further marker and/or parameter of said reference levels indicate that the risk of the subject to have (an) organ dysfunction(s) is decreased, and/or wherein higher or lower levels/values of said at least one further marker and/or parameter compared to the corresponding levels of said at least one further marker and/or parameter of said reference levels indicate that the risk to have (an) organ dysfunction(s) is increased.

Accordingly, the methods and kits of the present invention can also comprise determining at least one further marker and/or parameter in addition to the at least one histone and proADM.

As used herein, a parameter is a characteristic, feature, or measurable factor that can help in defining a particular system. A parameter is an important element for health- and physiology-related assessments, such as a disease/disorder/clinical condition risk, preferably organ dysfunction(s). Furthermore, a parameter is defined as a characteristic that is objectively measured and evaluated as an indicator of normal biological processes, pathogenic processes, or pharmacologic responses to a therapeutic intervention. An exemplary parameter can be selected from the group consisting of Acute Physiology and Chronic Health Evaluation score (APACHE scores I-IV), APACHE II, the simplified acute physiology score (SAPS I-III score), sequential organ failure assessment score (SOFA score), simplified acute physiology score II (SAPSII score) , mortality probability model (MPM I-III), multiple organ dysfunction score (MODS), therapeutic intervention scoring system (TISS), nine equivalents of nursing manpower use score (NEMS), World Federation of Neurosurgical Societies (WFNS) grading, and Glasgow Coma Scale (GCS), age, gender, family history, ethnicity, body weight, body mass index (BMI), cystoscopy report, white blood cell count, CT scan, blood pressure, heart rate, antihypertensive treatment, liquid intake, wheezing, body temperature, presence of diabetes mellitus and current smoking habits.

As used herein, terms such as "marker", "surrogate", "prognostic marker", "factor" or "biomarker" or "biological marker" are used interchangeably and relate to measurable and quantifiable biological markers (e.g., specific protein or enzyme concentration or a fragment thereof, specific hormone concentration or a fragment thereof, or presence of biological substances or a fragment thereof) which serve as indices for health- and physiology-related assessments, such as a disease/disorder/clinical condition risk, preferably an adverse event. A marker is defined as a characteristic that can be objectively measured and evaluated as an indicator of normal biological processes, pathogenic processes, or pharmacologic responses to a therapeutic intervention. Biomarkers may be measured in a sample (as a blood, plasma, urine, or tissue test). The at least one further marker of said subject can be selected from the group consisting of procalcitonin, calcitonin, Endothelin-1 (ET-1), Arginine Vasopressin (AVP), Atrial Natriuretic Peptide (ANP), Neutrophil Gelatinase-Associated Lipocalin (NGAL), Troponin, Brain Natriuretic Peptide (BNP), C-Reactive Protein (CRP), Pancreatic Stone Protein (PSP), Triggering Receptor Expressed on Myeloid Cells 1 (TREM1), Interleukin-6 (IL-6), Interleukin-1, Interleukin-24 (IL-24) other ILs, Presepsin (sCD14-ST), Lipopolysaccharide Binding Protein (LBP), Alpha-1-Antitrypsin, Matrix Metalloproteinase 2 (MMP2), Matrix Metalloproteinase 9 (MMP9), Matrix Metalloproteinase 7 (MMP9), Chromogranin A, S100A protein, S100B protein and Tumor Necrosis Factor α (TNFα) or a fragment thereof.

In particular aspects of the invention, the at least one further marker and/or parameter of said subject can be selected from the group consisting of aldolase B, copeptin, lactate, procalcitonin (PCT), the sequential organ failure assessment score (SOFA score), the simplified acute physiology score (SAPSII), heparin binding protein (HBP), the Acute Physiology and Chronic Health Evaluation II (APACHE II) score, and soluble fms-like tyrosine kinase-1 (sFlt-1). It is herein understood that particularly level(s) of the further marker(s) are determined in the sample of the subject.

As disclosed herein, the at least one further marker and/or parameter of said subject may be selected from the group consisting of procalcitonin, calcitonin, Endothelin-1 (ET-1), Arginine Vasopressin (AVP), Atrial Natriuretic Peptide (ANP), Neutrophil Gelatinase-Associated Lipocalin (NGAL), Troponin, Brain Natriuretic Peptide (BNP), C-Reactive Protein (CRP), Pancreatic Stone Protein (PSP), Triggering Receptor Expressed on Myeloid Cells 1 (TREM1), Interleukin-6 (IL-6), Interleukin-1, Interleukin-24 (IL-24) other ILs, Presepsin (sCD14-ST), Lipopolysaccharide Binding Protein (LBP), Alpha-1-Antitrypsin, Matrix Metalloproteinase 2 (MMP2), Matrix Metalloproteinase 9 (MMP9), Matrix Metalloproteinase 7 (MMP9), Chromogranin A, S100A protein, S100B protein, Tumor Necrosis Factor α (TNFα), age, gender, family history, ethnicity, body weight, body mass index (BMI), cystoscopy report, white blood cell count, CT scan, blood pressure, heart rate, antihypertensive treatment, liquid intake, wheezing, , body temperature, presence of diabetes mellitus, current smoking habits, Acute Physiology and Chronic Health Evaluation score (APACHE scores I-IV), the simplified acute physiology score (SAPS I-III score), sequential organ failure assessment score (SOFA score), IGS II, mortality probability model (MPM I-III), multiple organ dysfunction score (MODS), therapeutic intervention scoring system (TISS), nine equivalents of nursing manpower use score (NEMS), World Federation of Neurosurgical Societies (WFNS) grading, and Glasgow Coma Scale (GCS).

As used herein, "aldolase B" refers to fructose-bisphosphate aldolase B or liver-type aldolase that is one of three isoenzymes (A, B, and C) of the class I fructose 1,6-bisphosphate aldolase enzyme. The level of Aldolase B in the sample of the subject can be determined by mass spectrometry based methods.

"Copeptin" is also referred to as "CT-proAVP" or "C-terminal portion of vasopressin". Vasopressin is a powerful vasoconstrictor. The level of CT-proAVP can be measured in the plasma or serum of a subject by immunoassays as described below.

As used herein, "lactate" refers to the maximal lactate concentration measured in the blood. Normally, the lactate concentration is assessed daily or even more often. The lactate concentration in the blood can be determined by lactate oxidase spectrophotometric methods.

As used herein, "procalcitonin" or "PCT" relates to a peptide spanning amino acid residues 1-116, 2-116, 3-116 or fragments thereof. Thus the length of procalcitonin fragments is at least 12 amino acids, preferably more than 50 amino acids, more preferably more than 110 amino acids. PCT may comprise post-translational modifications such as glycosylation, liposidation or derivatisation. Procalcitonin is a precursor of calcitonin and katacalcin. Thus, under normal conditions the PCT levels in the circulation are very low (< about 0.05 ng/ml). The level of PCT in the sample of the subject can be determined by immunoassays as described below.

As used herein, the "sequential organ failure assessment score" or "SOFA score" is one score used to track a patient's status during the stay in an intensive care unit (ICU). The SOFA score is a scoring system to determine the extent of a person's organ function or rate of failure. The score is based on six different scores, one each for the respiratory, cardiovascular, hepatic, coagulation, renal and neurological systems. Both the mean and highest SOFA scores being predictors of outcome. An increase in SOFA score during the first 24 to 48 hours in the ICU predicts a mortality rate of at least 50% up to 95%. Scores less than 9 give predictive mortality at 33% while above 14 can be close to or above 95%.

As used herein, "SAPS II" or "Simplified Acute Physiology Score II" relates to a system for classifying the severity of a disease or disorder (see Le Gall JR et al., A new Simplified Acute Physiology Score (SAPS II) based on a European/North American multicenter study. JAMA. 1993;270(24):2957-63.). The SAPS II score is mainly made of 12 physiological variables and 3 disease-related variables. The point score is calculated from 12 routine physiological measurements, information about previous health status and some information obtained at admission to the ICU. The SAPS II score can be determined at any time, preferably, at day 2 after admission. The "worst" measurement is defined as the measure that correlates to the highest number of points. The SAPS II score ranges from 0 to 163 points. The classification system includes the followings parameters: Age, Heart Rate, Systolic Blood Pressure, Temperature, Glasgow Coma Scale, Mechanical Ventilation or CPAP, PaO2, FiO2, Urine Output, Blood Urea Nitrogen, Sodium, Potassium, Bicarbonate, Bilirubin, White Blood Cell, Chronic diseases and Type of admission. There is a sigmoidal relationship between mortality and the total SAPS II score. The mortality of a subject is 10% at a SAPSII score of 29 points, the mortality is 25% at a SAPSII score of 40 points, the mortality is 50% at a SAPSII score of 52 points, the mortality is 75% at a SAPSII score of 64 points, the mortality is 90% at a SAPSII score of 77 points (Le Gall loc. cit.).

As used herein, "heparin-binding protein" or "HBP", refers to a protein released from activated neutrophils, which is a potent inducer of vascular leakage. The level of HBP in the sample of the subject can be determined by mass spectrometer based assays as described herein below.

As used herein, "APACHE II" or "Acute Physiology and Chronic Health Evaluation II" is a severity-of-disease classification scoring system (Knaus et al., 1985). It can be applied within 24 hours of admission of a patient to an intensive care unit (ICU) and may be determined based on e.g. 12 or 14 different physiologic parameters.

As used herein, "soluble fms-like tyrosine kinase-1" or "sFlt-1" is a tyrosine kinase protein that disables proteins that cause blood vessel growth. Soluble Flt-1 (sFlt-1) is a splice variant of VEGF receptor 1 (Flt-1) which is produced by a variety of tissues. The level of sFLT1 in the sample of the subject can be determined by mass spectrometry based assays or by an immunoassay.

The methods or the kits of the invention can comprise determining the level of at least one histone and the level of proADM and a level of a further marker and/or parameter as described above.

For example, the method can comprise determining the level of at least one histone and the level of proADM in the sample of the subject and the SOFA score of the subject.

Further, the method can comprise determining the level of at least one histone and the level of proADM in the sample of the subject and the SAPS II score of the subject.

Further, the method can comprise determining the level of at least one histone and the level of proADM in the sample of the subject and the level of aldolase B in the sample of the subject.

Further, the method can comprise determining the level of at least one histone and the level of proADM in the sample of the subject and the level of PCT in the sample of the subject.

Further, the method can comprise determining the level of at least one histone and the level of proADM in the sample of the subject and the level of lactate in the sample of the subject.

Further, the method can comprise determining the level of at least one histone and the level of proADM in the sample of the subject and the level of copeptin in the sample of the subject.

Particularly, the method can comprise determining the level of histone H2B in said sample and the SOFA score of said subject. Particularly, the method can comprise determining the level of histone H4 in said sample and the SOFA score of said subject.

Particularly, the method can comprise determining the level of histone H2A in said sample and the SOFA score of said subject. Particularly, the method can comprise determining the level of histone H2A in said sample and the SAPSII score of said subject. Particularly, the method can comprise determining the level of histone H4 in said sample and the SAPSII score of said subject. Particularly, the method can comprise determining the level of histone H2B in said sample and the SAPSII score of said subject.

Particularly, the method can comprise determining the level of proADM in said sample and said level of aldolase B in said sample. Particularly, the method can comprise determining the level of proADM in said sample and said level of H2B in said sample. Particularly, the method can comprise determining the level of proADM in said sample and said level of H2A in said sample. Particularly, the method can comprise determining the level of proADM in said sample and said level of H4 in said sample. Particularly, the method can comprise determining the level of proADM in said sample and said level of lactate in said sample. Particularly, the method can comprise determining the level of proADM in said sample and said level of H3 in said sample.

As used herein, the "subject" (or "patient") may be a vertebrate As used herein, the term "subject" may include both humans and animals, particularly mammals, and other organisms. Thus, the herein provided methods are applicable to both human and animal subjects. Accordingly, said subject may be an animal such as a mouse, rat, hamster, rabbit, guinea pig, ferret, cat, dog, chicken, sheep, bovine species, horse, camel, or primate. Preferably, the subject is a mammal. Most preferably, the subject is human.

As it is shown in the appended examples, the organ dysfunction of subjects suffering from various disorders or diseases is predicted. Therefore, the method provided herein can be used on any subject that is a healthy subject or a subject that suffers from any disease or disorder. In preferred aspects, the subject suffers from a disease, disorder or medical condition. The subject to be tested can be a critical ill patient, preferably wherein said subject is admitted to an emergency department or wherein said subject is admitted to an intensive care unit.

The subject to be tested can be a subject that suffers from a disease or medical condition and wherein said disease or medical condition is selected from the group consisting of cardiovascular disease, diabetes mellitus, malignancy, respiratory disease, liver disease, renal disease immunodepression, an inflammatory response related to infective and non-infective etiologies, systemic inflammatory response syndrome (SIRS), sepsis, severe sepsis, and/or septic shock.

Particularly, the subject suffers from sepsis. More particularly, the subject suffers from severe sepsis and/or septic shock.

"Systematic inflammation" may preferably relate to a condition characterized by a release of pro-inflammatory cytokines and an activated innate immune system which can be caused by biological factors, chemical factors or by genetic factors. Severe "Systemic Inflammation" can lead to organ failure and death.

"SIRS" in the context of the invention is a systemic inflammatory response syndrome with no signs of infection. It includes, but is not limited to more than one of the following clinical manifestations: (1) a body temperature greater than 38°C or less than 36°C; (2) a heart rate greater than 90 beats per minute; (3) tachypnea, manifested by a respiratory rate greater than 20 breaths per minute, or hyperventilation, as indicated by a PaCO₂ of less than 32 mm Hg; and (4) an alteration in the white blood cell count such as a count greater than 12,000/mm³, a count less than 4,000/mm³, or the presence of more than 10% immature neutrophiles (Bone et al., CHEST 101(6): 1644-55, 1992).

"Sepsis" in the context of the invention refers to a systemic response to infection. Alternatively, sepsis may be seen as the combination of SIRS with a confirmed infectious process or an infection. Sepsis may be characterized as clinical syndrome defined by the presence of both infection and a systemic inflammatory response (Levy MM et al. 2001 SCCM/ESICM/ACCP/ATS/SIS International Sepsis Definitions Conference. Crit Care Med. 2003 Apr;31(4):1250-6). The term "sepsis" used herein includes, but is not limited to, sepsis, severe sepsis, septic shock. Severe sepsis in this context means sepsis associated with organ dysfunction, hypoperfusion abnormality, or sepsis-induced hypotension. Hypoperfusion abnormalities include lactic acidosis, oliguria and acute alteration of mental status. Sepsis-induced hypotension is defined by the presence of a systolic blood pressure of less than about 90 mm Hg or its reduction by about 40 mm Hg or more from baseline in the absence of other causes for hypotension (e.g. cardiogenic shock). Septic shock is defined as severe sepsis with sepsis-induced hypotension persisting despite adequate fluid resuscitation, along with the presence of hypoperfusion abnormalities or organ dysfunction (Bone et al., CHEST 101(6): 1644-55, 1992).

The term "sepsis" used herein relates to all possible stages in the development of sepsis.

As used herein, "infection" may mean a pathological process caused by the invasion of normally sterile tissue or fluid by pathogenic or potentially pathogenic microorganisms and relates to infection(s) by bacteria, viruses, fungi, and/or parasites. Accordingly, the infection can be a bacterial infection, viral infection, and/or fungal infection. The infection can be a local or systemic infection. Further, the subject suffering from an infection can suffer from more than one source(s) of infection simultaneously. For example, the subject suffering from an infection can suffer from a bacterial infection and viral infection; from a viral infection and fungal infection; from a bacterial and fungal infection, and from a bacterial infection, fungal infection and viral infection.

As used herein, the term "sample" is a biological sample that is obtained from the subject. The term "sample of the subject" and "sample from the subject" can be used interchangeably herein. The samples could be processed (pre-treated), such as by fractionation or purification procedures, for example, separation of whole blood into serum or plasma components. Such pre-treatments can also include, but are not limited to dilution, filtration, centrifugation, concentration, sedimentation, precipitation or dialysis. Pre-treatments may also include the addition of chemical or biochemical substances to the solution, such as acids, bases, buffers, salts, solvents, reactive dyes, detergents, emulsifiers, chelators. The sample is a blood sample, more preferably a serum sample or a plasma sample.

"Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood), for example for at least 15 minutes at 2000 to 3000 g.

"Serum" in the context of the present invention is the liquid fraction of whole blood that is collected after the blood is allowed to clot. When coagulated blood (clotted blood) is centrifuged serum can be obtained as supernatant.

As used herein, "urine" is a liquid product of the body secreted by the kidneys through a process called urination (or micturition) and excreted through the urethra.

As described above, the level of at least one histone and/or of proADM is determined in the sample of the subject. The skilled person is aware of methods/assay that can be employed to determine the level of biomarkers in a sample.

As described above, the level of at least one histone is determined, particularly, the level(s) of the histones H2B, H4, H2A and/or H3 is/are determined. Particularly, the histone or the histone fragment can be determined. Such fragments are herein exemplified below.

Such a histone or fragment thereof may also represent a free histone. For example, the methods and kits of the present invention may particularly detect peptides or epitopes of free histones. Such stretches of amino acids are also referred herein as central regions or parts of the histones. In the following, peptides or epitopes are described that may also be employed to detect free histones using the methods herein provided.

In particular, the at least one histone can be histone H4 and wherein at least a peptide of the sequence spanning amino acid residues 22 to 102 of histone H4 according to SEQ ID NO:1 is determined. Particularly, the least one histone is histone H4 and wherein at least a peptide of the sequence is determined selected from the group consisting of an amino acid sequence spanning residues 46 to 56 of SEQ ID NO:1, residues 67 to 78 of SEQ ID NO: 1, residues 60 to 67 of SEQ ID NO: 1, residues 22 to 30 of SEQ ID NO: 1, residues 67 to 78 of SEQ ID NO: 1, residues 92 to 102 of SEQ ID NO: 1, residues 22 to 34 of SEQ ID NO: 1, residues 46 to 102 of SEQ ID NO: 1, residues 46 to 55 of SEQ ID NO: 1, residues 80 to 91 of SEQ ID NO: 1, residues 24 to 35 of SEQ ID NO: 1, and residues 68 to 77 of SEQ ID NO: 1. For example, two peptides may be determined.

Particularly, the least one histone is histone H4 and wherein the peptides are determined of an amino acid sequence spanning residues 46 to 56 of SEQ ID NO:1 and residues 67 to 78 of SEQ ID NO: 1.

Further, the least one histone is histone H2A and wherein at least a peptide of the sequence spanning amino acid residues 20 to 118 of histone H2A according to SEQ ID NO: 2 is determined. In particular, the least one histone is histone H2A and wherein at least a peptide of the sequence is determined selected from the group consisting of an amino acid sequence spanning residues 21 to 53 of SEQ ID NO: 2, residues 21 to 29 of SEQ ID NO: 2, residues 30 to 53 of SEQ ID NO: 2, residues 120 to 129 of SEQ ID NO: 2, residues 21 to 29 of SEQ ID NO: 2, residues 82 to 88 of SEQ ID NO: 2, residues 89 to 95 of SEQ ID NO: 2, and residues 100 to 118 of SEQ ID NO: 2.

Further, the least one histone is histone H3 and wherein at least a peptide of the sequence spanning amino acid residues 27 to 62 of histone H3 according to SEQ ID NO: 3 is determined. Further, the least one histone is histone H3 and wherein at least a peptide of the sequence spanning amino acid residues 27 to 37 of SEQ ID NO: 3 and/or spanning amino acid residues 52 to 62 of SEQ ID NO: 3 is determined.

Further, the least one histone is histone H2B and wherein at least a peptide of the sequence spanning amino acid residues 41 to 69 of histone H2B according to SEQ ID NO: 4 is determined.

Further, the least one histone is histone H2A and wherein at least a peptide or a fragment thereof is determined selected from the group consisting of SEQ ID NOs: 7, 8, 9 and 10 is determined.

Further, the least one histone is histone H4 and wherein at least a peptide or a fragment thereof selected from the group consisting of SEQ ID NOs: 11, 12, 13, 14, 15 and 16 is determined.

It is herein understood that one, two three, four or more peptides can be determined.

The level of the markers, e.g. the at least one histone or the fragment thereof and/or the proADM or the fragment thereof, can be determined by any assay that reliably determines the concentration of the marker. Particularly, mass spectrometry (MS) and/or immunoassays can be employed as exemplified in the appended examples. As used herein, an immunoassay is a biochemical test that measures the presence or concentration of a macromolecule/polypeptide in a solution through the use of an antibody or antibody binding fragment or immunoglobulin.

Alternatively, instead of antibodies, other capture molecules or molecular scaffolds that specifically and/or selectively recognize histone sequences, histone epitopes, and structural conformations of histones may be used . Herein, the term "capture molecules" or "molecular scaffolds" comprises molecules which may be used to bind target molecules or molecules of interest, i.e. analytes (e.g. the histone(s) and/or proADM), from a sample. Capture molecules must thus be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may, for instance, be mediated by ionic, van-der-Waals, pi-pi, sigma-pi, hydrophobic or hydrogen bond interactions or a combination of two or more of the aforementioned interactions or covalent interactions between the capture molecules or molecular scaffold and the target molecules or molecules of interest. Capture molecules or molecular scaffolds may for instance be selected from the group consisting of a nucleic acid molecule, a carbohydrate molecule, a PNA molecule, a protein, a peptide and a glycoprotein. Capture molecules or molecular scaffolds include, for example, aptamers, DARpins (Designed Ankyrin Repeat Proteins). Affimers and the like.

As used herein, the term, "antibody" refers to immunoglobulin molecules and immunologically active portions of immunoglobulin (Ig) molecules, i.e., molecules that contain an antigen binding site that specifically binds (immuno reacts with) an antigen. The antibodies may be monoclonal as well as polyclonal antibodies. Particularly, antibodies that are specifically binding to the at least one histone and/or that bind specifically to proADM are used. An antibody is considered to be specific, if its affinity towards the molecule of interest, e.g. the at least one histone and/or proADM, or the fragment thereof is at least 50-fold higher, preferably 100-fold higher, most preferably at least 1000-fold higher than towards other molecules comprised in a sample containing the molecule of interest. It is well known in the art how to develop and to select antibodies with a given specificity. Monoclonal antibodies may be preferred. The antibody or the antibody binding fragment binds specifically to the herein defined markers or fragments thereof. In particular, the antibody or the antibody binding fragment binds to the herein defined peptides of the at least one histone protein. Thus, the herein defined peptides can also be epitopes to which the antibodies specifically bind to. Further, an antibody or an antibody binding fragment may be used in the methods and kits of the invention as defined in the claims that binds specifically to proADM, particularly to MR-proADM. Exemplary immunoassays can be luminescence immunoassay (LIA), radioimmunoassay (RIA), chemiluminescence- and fluorescence- immunoassay, enzyme immunoassay (EIA), Enzyme-linked immunosorbent assays (ELISA), luminescence-based bead assay, magnetic beads based assay, protein microarray assay, rapid test formats or rare cryptate assay. Further, assays suitable for point-of-care testing and rapid test formats such as for instance immune-chromatographic strip tests can be employed.

In certain aspects of the invention as defined in the claims, the method is a method for the diagnosis and/or monitoring of the number of organ failures in a subject, wherein said method comprises
(i) obtaining a sample of the subject;
(ii) detecting a level of at least one histone and a level of proadrenomedullin (proADM) in the sample of said subject by contacting the sample with antibodies or antigen-binding fragments or derivatives thereof specific for an epitope of said at least one histone and of said proADM and detecting binding between the antibodies or the antigen-binding fragments or derivatives thereof and said at least one histone and said proADM; and
(iii) wherein said level of at least one histone and said level of proadrenomedullin (proADM) are indicative of said organ dysfunction.

In certain aspects of the invention as defined in the claims, the method is an immunoassay comprising the steps of:
a) contacting the sample with
   (i) a first antibody or an antigen-binding fragment or derivative thereof specific for a first epitope of a histone or of proADM, and
   (ii) a second antibody or an antigen-binding fragment or derivative thereof specific for a second epitope of the histone or the proADM; and
b) detecting the binding of the first and second antibodies or antigen-binding fragments or derivates thereof to the histone or to proADM.

Preferably, one of the antibodies can be labeled and the other antibody can be bound to a solid phase or can be bound selectively to a solid phase. In a particularly preferred aspect of the assay, one of the antibodies is labeled while the other is either bound to a solid phase or can be bound selectively to a solid phase. The first antibody and the second antibody can be present dispersed in a liquid reaction mixture, and wherein a first labelling component which is part of a labelling system based on fluorescence or chemiluminescence extinction or amplification is bound to the first antibody, and a second labelling component of said labelling system is bound to the second antibody so that, after binding of both antibodies to said at least one histone and/or to said proADM to be detected, a measurable signal which permits detection of the resulting sandwich complexes in the measuring solution is generated. The labelling system can comprise a rare earth cryptate or chelate in combination with a fluorescent or chemiluminescent dye, in particular of the cyanine type.

The method may be executed as heterogeneous sandwich immunoassay, wherein one of the antibodies is immobilized on an arbitrarily chosen solid phase, for example, the walls of coated test tubes (e.g. polystyrol test tubes; coated tubes; CT) or microtiter plates, for example composed of polystyrol, or to particles, such as for instance magnetic particles, whereby the other antibody has a group resembling a detectable label or enabling for selective attachment to a label, and which serves the detection of the formed sandwich structures. A temporarily delayed or subsequent immobilization using suitable solid phases is also possible.

The method as disclosed herein may furthermore be embodied as a homogeneous method, wherein the sandwich complexes formed by the antibody/antibodies and the marker, e.g., the histone or the proADM or a fragment thereof, which is to be detected remains suspended in the liquid phase. In this case it is preferred, that when two antibodies are used, both antibodies are labeled with parts of a detection system, which leads to generation of a signal or triggering of a signal if both antibodies are integrated into a single sandwich. Such techniques are to be embodied in particular as fluorescence enhancing or fluorescence quenching detection methods. A particularly preferred aspect relates to the use of detection reagents which are to be used pair-wise, such as for example the ones which are described in US 4 882 733 A, EP-B1 0 180 492 or EP-B1 0 539 477 and the prior art cited therein. In this way, measurements in which only reaction products comprising both labeling components in a single immune-complex directly in the reaction mixture are detected, become possible. For example, such technologies are offered under the brand names TRACE^{®} (Time Resolved Amplified Cryptate Emission) or KRYPTOR^{®}, implementing the teachings of the above-cited applications. Therefore, in particular preferred aspects, a diagnostic device is used to carry out the herein provided method. For example, the level of the histone or proADM or a fragment thereof, and/or the level of any further marker of the herein provided method is determined. In particular preferred aspects, the diagnostic device is KRYPTOR^{®}.

Further, the immunoassay methods of the present invention as defined in the claims may preferably utilize a first antibody and/or a second antibody or antigen-binding fragment(s) or derivative(s) thereof being specific for (an) epitope(s) of at least one histone and/or of proADM. Exemplary, peptides are described herein below and above that can be suitable for the determination of the level of proADM and/or of the at least one histone.

For example, the immunoassay methods of the present invention as defined in the claims may preferably utilize a first antibody and/or a second antibody or antigen-binding fragment(s) or derivative(s) thereof being specific for (an) epitope(s) of histone H4, wherein the first epitope and/or second epitope are epitopes of histone H4 present in the sequence spanning amino acid residues 22 to 102 of SEQ ID NO:1.

Further, the immunoassay methods of the present invention as defined in the claims may utilize a first antibody, antigen-binding fragment or derivative thereof that is specific for an epitope of histone H4 present in the sequence spanning amino acid residues 46 to 56 of SEQ ID NO:1, and a second antibody, antigen-binding fragment or derivative thereof that is specific for an epitope of histone H4 present in the sequence spanning amino acid residues 67 to 78 of SEQ ID NO: 1.

For example, the immunoassay methods of the present invention as defined in the claims may preferably utilize a first antibody and/or a second antibody or antigen-binding fragment(s) or derivative(s) thereof being specific for (an) epitope(s) of histone H2B, wherein the first epitope and/or second epitope are epitopes of histone H2B present in the sequence spanning amino acid residues 41 to 69 of SEQ ID NO:4.

Further, the immunoassay methods of the present invention as defined in the claims may preferably utilize a first antibody and/or a second antibody or antigen-binding fragment(s) or derivative(s) thereof being specific for (an) epitope(s) of histone H2A, wherein the first epitope and/or second epitope are epitopes of histone H2A present in the sequence spanning amino acid residues 20 to 118 of SEQ ID NO:2.

Further, the immunoassay methods of the present invention as defined in the claims may utilize a first antibody and/or a second antibody or antigen-binding fragment(s) or derivative(s) thereof being specific for (an) epitope(s) of histone H2A, wherein the first epitope and/or second epitope are epitopes of histone H2A present in the sequence spanning amino acid residues 21 to 53, 20 to 118 or 120 to 129 of SEQ ID NO:2.

Further, the immunoassay methods of the present invention as defined in the claims may utilize a first antibody and/or a second antibody or antigen-binding fragment(s) or derivative(s) thereof being specific for (an) epitope(s) of histone H3, wherein the first epitope and/or second epitope are epitopes of histone H3 present in the sequence spanning amino acid residues 27 to 62 of SEQ ID NO:3.

More preferably, the immunoassay methods of the present invention as defined in the claims may utilize a first antibody and/or a second antibody or antigen-binding fragment(s) or derivative(s) thereof being specific for (an) epitope(s) of histone H4, wherein the epitope(s) is/are selected from the group consisting of an amino acid sequence spanning residues 22 to 30 of SEQ ID NO:1, residues 46 to 56 of SEQ ID NO:1, residues 67 to 78 of SEQ ID NO:1, residues 92 to 102 of SEQ ID NO:1, residues 22 to 34 of SEQ ID NO: 1, and residues 46 to 102 of SEQ ID NO: 1.

Further, the immunoassay methods of the present invention as defined in the claims may utilize a first antibody and/or a second antibody or antigen-binding fragment(s) or derivative(s) thereof being specific for (an) epitope(s) of histone H2A, wherein the epitope(s) is/are selected from the group consisting of an amino acid sequence spanning residues 21 to 53 of SEQ ID NO:2, residues 21 to 29 of SEQ ID NO:2, residues 30 to 53 of SEQ ID NO:2, and residues 120 to 129 of SEQ ID NO: 2.

Further, the immunoassay methods of the present invention as defined in the claims may utilize a first antibody and/or a second antibody or antigen-binding fragment(s) or derivative(s) thereof being specific for (an) epitope(s) of histone H2B spanning residues 41 to 69 of SEQ ID NO: 4.

Further, the immunoassay methods of the present invention may utilize a first antibody and/or a second antibody or antigen-binding fragment(s) or derivative(s) thereof being specific for (an) epitope(s) of histone H3 spanning residues 27 to 37 of SEQ ID NO: 3 and/or spanning residues 52 to 62 of SEQ ID NO: 3.

Further, the immunoassay methods of the present invention as defined in the claims may utilize a first antibody and/or the second antibody or the antigen-binding fragment or derivative thereof which are specific for an epitope of histone H2A present in the sequence spanning amino acid residues 21 to 53 and/or 120 to 129 of the histone H2A sequence represented by SEQ ID NO:2. Further, the immunoassay methods of the present invention as defined in the claims may utilize a first antibody, antigen-binding fragment or derivative thereof that is specific for an epitope of histone H4 present in the sequence spanning amino acid residues 22 to 102 of SEQ ID NO:1, and a second antibody, antigen-binding fragment or derivative thereof that is specific for an epitope of a free histone H2A, H2B, or preferably H3.

Further, the immunoassay methods of the present invention as defined in the claims may utilize a first antibody, antigen-binding fragment or derivative thereof that is specific for an epitope of histone H2B present in the sequence spanning amino acid residues 41 to 69 of SEQ ID NO:4, and a second antibody, antigen-binding fragment or derivative thereof that is specific for an epitope of a free histone H2A, H4, or H3.

Further, the immunoassay methods of the present invention as defined in the claims may utilize a first antibody, antigen-binding fragment or derivative thereof that is specific for an epitope of histone H2B present in the sequence spanning amino acid residues 20 to 118 of SEQ ID NO:2, and a second antibody, antigen-binding fragment or derivative thereof that is specific for an epitope of a free histone H2B, H4, or H3.

Further, the immunoassay methods of the present invention as defined in the claims may utilize a first antibody, antigen-binding fragment or derivative thereof that is specific for an epitope of histone H2B present in the sequence spanning amino acid residues 27 to 62 of SEQ ID NO:3, and a second antibody, antigen-binding fragment or derivative thereof that is specific for an epitope of a free histone H2B, H4, or H2A.

Further, the immunoassay methods of the present invention as defined in the claims may utilize a first antibody, antigen-binding fragment or derivative thereof that is specific for an epitope of histone H2A present in the sequence spanning amino acid residues 21 to 53, 120 to 129, or 20 to 118 of SEQ ID NO:2, and a second antibody, antigen-binding fragment or derivative thereof that is specific for an epitope of a free histone H3, H4 or preferably H2B.

Further disclosed herein is an antibody or an antigen-binding fragment or derivative thereof which is specific for an epitope of a histone protein or fragment thereof as detailed above. Exemplary antibodies that are successfully employed to detect histones or proADM, preferably MR-proADM are shown in the appended examples. The present invention as defined in the claims thus relates to an antibody(ies), (an) antigen-binding fragment(s) or derivative(s) thereof that is/are specific for an epitope of histone H2B, H4, H2A, H3 and/or proADM, preferably MR-proADM. Exemplary epitopes or peptides to which the antibodies are specifically binding to are herein documented above and below.

The level of the marker, e.g. the at least one histone and/or proADM, can also be determined by a mass spectrometric (MS) based analysis as described in the appended examples. Such a method may comprise detecting the presence, amount or concentration of one or more modified or unmodified fragment peptides of e.g. proADM and/or the histone in said biological sample or a protein digest (e.g. tryptic digest) from said sample, and optionally separating the sample with chromatographic methods, and subjecting the prepared and optionally separated sample to MS analysis. For example, selected reaction monitoring (SRM), multiple reaction monitoring (MRM) or parallel reaction monitoring (PRM) mass spectrometry may be used in the MS analysis, particularly to determine the amounts of at least one histone peptide. Herein, the term "mass spectrometry" or "MS" refers to an analytical technique to identify compounds by their mass. In order to enhance the mass resolving and mass determining capabilities of mass spectrometry, the samples can be processed prior to MS analysis. Accordingly, the invention as defined in the claims relates to MS detection methods that can be combined with immuno-enrichment technologies, methods related to sample preparation and/or chromatographic methods, preferably with liquid chromatography (LC), more preferably with high performance liquid chromatography (HPLC) or ultra high performance liquid chromatography (UHPLC). Sample preparation methods comprise techniques for lysis, fractionation, digestion of the sample into peptides, depletion, enrichment, dialysis, desalting, alkylation and/or peptide reduction. However, these steps are optional. The selective detection of analyte ions may be conducted with tandem mass spectrometry (MS/MS). Tandem mass spectrometry is characterized by mass selection step (as used herein, the term "mass selection" denotes isolation of ions having a specified m/z or narrow range of m/z's), followed by fragmentation of the selected ions and mass analysis of the resultant product (fragment) ions.

The skilled person is aware how quantify the level of a marker in the sample by mass spectrometric methods. For example, relative quantification "rSRM" or absolute quantification can be employed as described above.

As used herein, "diagnosis" in the context of the present invention relates to the recognition and (early) detection of organ failure in a subject and may also comprise differential diagnosis. Also the assessment of the severity of the organ dysfunction may be encompassed by the term "diagnosis". For example, the assessment of how many organ dysfunctions and/or organ failures the subject may suffer from.

"Prognosis" relates to the prediction of an outcome or a specific risk for a subject to suffer from an organ dysfunction and/or organ failure. This may also include an estimation of the chance of recovery or the chance of an adverse outcome for said subject.

The methods of the invention may also be used for monitoring. "Monitoring" relates to keeping track of an already diagnosed organ dysfunction and/or organ failure, disease, disorder, complication or risk, e.g. to analyze the progression of the disease or the influence of a particular treatment on the progression of the organ dysfunction and/or organ failure, disease or disorder.

The term "therapy control" may refer to the monitoring and/or adjustment of a therapeutic treatment of a subject.

The terms "risk assessment" and "risk stratification" may relate to the grouping of subjects into different risk groups according to their further prognosis. Risk assessment also relates to stratification for applying preventive and/or therapeutic measures.

The term "therapy guidance" may refer to application of certain therapies or medical interventions based on the value of one or more biomarkers and/or clinical parameter and/or clinical scores.

The invention further relates to kits, the use of the kits and methods wherein such kits are used. The invention relates to kits for carrying out the herein above and below provided methods. The herein provided definitions, e.g. provided in relation to the methods, also apply to the kits of the invention. In particular, the invention relates to kits for carrying out the method according to the invention, wherein said kit comprises
(i) detection reagents for determining said level of at least one histone in a sample from said subject, comprising a ligand which specifically binds to said at least one histone, and
   reference data including a reference level of at least one histone, and wherein an increased level of the at least one histone in the sample as compared to said reference level of the at least one histone is indicative of organ dysfunction in said subject; and
(ii) detection reagents for determining said level of proADM in said sample, comprising a ligand which specifically binds to said proADM, and
   reference data including a reference level of proADM, and
   wherein an increased level of proADM in the sample as compared to the reference level of proADM is indicative of organ dysfunction in said subject.

As used herein, "reference data" comprise reference level(s) of at least one histone and/or of proADM, particularly MR-proADM. The levels of the at least one histone and/or of proADM in the sample of the subject can be compared to the reference levels comprised in the reference data of the kit. An increased level of the marker(s) determined is indicative of organ dysfunction. The reference levels are herein described above and are exemplified also in the appended examples. The reference data can also include a reference sample to which the level of the at least one histone and/or the level of proADM is compared to. The reference data can also include an instruction manual how to use the kits of the invention.

As used herein, the "detection reagent" or the like are reagents that are ligands that specifically bind to the at least one histone and to proADM. Such ligands might be used in immunoassays as described above. Further reagents that are employed in the immunoassays to determine the level of the marker(s) may also be comprised in the kit and are herein considered as detection reagents. Further detection reagents can also relate to reagents that are employed to detect the markers or fragments thereof by MS based methods. Such detection reagent can thus also be reagents, e.g. enzymes, chemicals, buffers, etc, that are used to prepare the sample for the MS analysis. A mass spectrometer can also be considered as a detection reagent. Further detection reagents according to the invention as defined in the claims can also be calibration solution(s), e.g. that can be employed to determine and compare the level of the marker(s).

As used herein, the terms "comprising" and "including" or grammatical variants thereof are to be taken as specifying at least the stated features, integers, steps or components but do not preclude the addition of one or more additional features, integers, steps, components or groups thereof. This term encompasses the terms "consisting of" and "consisting essentially of" that are understood to specify only the stated feature, integers, steps or components to the exclusion of any additional features.

Thus, the terms "comprising"/"including"/"having" mean that any further component (or likewise features, integers, steps and the like) can/may be present.

The term "consisting of" means that no further component (or likewise features, integers, steps and the like) is present.

The term "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

Thus, the term "consisting essentially of" means those specific further components (or likewise features, integers, steps and the like) can be present, namely those not materially affecting the essential characteristics of the composition, device or method. In other words, the term "consisting essentially of" (which can be interchangeably used herein with the term "comprising substantially"), allows the presence of other components in the composition, device or method in addition to the mandatory components (or likewise features, integers, steps and the like), provided that the essential characteristics of the device or method are not materially affected by the presence of other components.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts.

The term "about" preferably refers to ±10% of the indicated numerical value, more preferably to ±5% of the indicated numerical value, and in particular to the exact numerical value indicated.

As used herein, the term "about" refers to ±10% of the indicated numerical value, and in particular to ±5% of the indicated numerical value. Whenever the term "about" is used, a specific reference to the exact numerical value indicated is also included. If the term "about" is used in connection with a parameter that is quantified in integers, such as the number of nucleotides in a given nucleic acid, the numbers corresponding to ±10% or ±5% of the indicated numerical value are to be rounded to the nearest integer. For example, the expression "about 25 amino acids" refers to the range of 23 to 28 amino acids, in particular the range of 24 to 26 amino acids, and preferably refers to the specific value of 25 amino acids.

The sensitivity and specificity of a diagnostic and/or prognostic test depends on more than just the analytical "quality" of the test. Sensitivity and specificity also depend on the definition of what constitutes an abnormal result. In practice, Receiver Operating Characteristic curves (ROC curves), are typically calculated by plotting the value of a variable versus its relative frequency in "normal" (i.e. apparently healthy individuals not having a prenatal disorder or condition) and "disease" populations, e.g. subjects having one or more organ dysfunction(s) or failure(s). For any particular marker (like MR-proADM), a distribution of marker levels for subjects with and without a disease/condition will likely overlap. Under such conditions, a test does not absolutely distinguish normal from disease with 100% accuracy, and the area of overlap might indicate where the test cannot distinguish normal from disease. A threshold is selected, below which the test is considered to be abnormal and above which the test is considered to be normal or below or above which the test indicates a specific condition, e.g. organ dysfunction. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art; see, e.g., Hanley et al. 1982. Radiology 143: 29-36. Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

A positive likelihood ratio, negative likelihood ratio, odds ratio, or hazard ratio may be used as a measure of a test's ability to predict risk or diagnose a disorder or condition ("diseased group").

In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group.

The skilled artisan will understand that associating a diagnostic or prognostic indicator, with a diagnosis or with a prognostic risk of a future clinical outcome is a statistical analysis. For example, a marker level of lower than X may signal that a patient is more likely to suffer from an adverse outcome than patients with a level more than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value; see, e.g., Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

Unless otherwise indicated, established methods of recombinant gene technology were used as described, for example, in Sambrook, Russell "Molecular Cloning, A Laboratory Manual", Cold Spring Harbor Laboratory, N.Y. (2001).

### Description of the Figure

**Figure 1****: Distribution of marker levels and clinical scores with respect to the number of OFs.** Boxplots illustrate the marker level (histone H2A (A); histone H2B (B); histone H3 (C); histone H4 (D); and MR-proADM (E)) or illustrate the score ((F): SOFA score) for each group represented by the number of OFs. The number of patients in each group is indicated in brackets (n).

### Example 1

### METHODS

### Study population

Two hundred and thirty-seven critically ill patients admitted to the medical intensive care unit (ICU) of 'centre hospitalier universitaire (CHU) de Dijon Bourgogne' from the 1^{st} of June 2013 to the 14^{th} of June 2014 were consecutively enrolled in the clinical study. Patients younger than 18 years were excluded. The study was approved by the local institutional review board. Before enrollment, written informed consent was obtained from patients themselves or from the patient's next of kin. All patients showed a broad spectrum of diseases including cardiovascular disease, diabetes mellitus, malignancy, respiratory disease, liver disease, renal disease and immunodepression and were monitored until discharge or death in the hospital. Based on retrospective review of medical records, imaging and microbiology results two independent physicians classified the patients on the day of admission as either non-sepsis (systemic inflammatory response syndrome (SIRS) or no SIRS, severe sepsis or septic shock according to international standardized criteria (Bone, Balk et al. 1992). A blood sample was taken on the day of admission, i.e. during the first 24 hours. Baseline demographics and clinical data including medical history, results from physical examination, routine blood analyses (e.g. blood cultures), non-laboratory diagnostic investigations (e.g. SIRS criteria, organ failure (OF) criteria), therapeutic interventions (e.g. mechanical ventilation (MV), vasopressors and renal replacement therapy (RRT)) as well as outcome parameters (e.g. length of stay, all cause mortality) were recorded. The sequential organ failure assessment (SOFA) score, based on six organ parameters, and the simplified acute physiology score (SAPS II), based on 17 mainly physiology variables, were calculated on admission (Le Gall, Lemeshow et al. 1993; Vincent, Moreno et al. 1996).

Seven types of acute organ failures were recorded at admission, for example circulatory shock (i), hematologic failure (ii), liver failure (iii), neurologic failure (iv), renal failure (v) as well as respiratory failure (vi) and metabolic acidosis (vii). The criteria for each single organ failure were employed as follows. For example, circulatory shock was defined as systolic arterial pressure lower than 90 mmHg with signs of peripheral hypoperfusion or the need for continuous infusion of vasopressor or inotropic agents. Hematologic failure was defined as thrombocythemia lower than 100,000/mm³. Liver failure was defined as bilirubinemia greater than 2 mg/dL and/or enzyme levels of aspartate or alanine transaminase greater than 500 international units per liter. Neurologic failure was defined as the Glasgow coma scale below 13. Renal failure was defined as urine output less than 0.5 ml/kg/h for at least 3 hours and/or creatinemia rising more than 50 % as compared to previous values. Respiratory failure was defined as the ratio of the partial pressure of arterial oxygen to the fraction of inspired oxygen (Pa O₂/Fi O₂) lower than 300 mmHg regardless of the chosen ventilatory support. Metabolic acidosis was defined as lactate levels below 2.5 mmol/L, as base excess (base level below -2 mEquivalent/L) or bicarbonate levels (HCO₃⁻) below 18 mmol/L.

### Biomarkers

MR-proADM (midregional proadrenomedullin), copeptin and PCT (procalcitonin) levels were determined in plasma samples using ultrasensitive assays, such as those developed for use with the KRYPTOR random access analyser (Thermo Scientific B·R·A·H·M·S). The levels of histone H2A, H2B, H3 and H4 as well as the level of Aldolase B were determined in the plasma samples by e.g. selected reaction monitoring or multiple reaction monitoring (SRM/MRM) assays as described in the following. Specific peptides derived from the markers were measured by LC-MS/MS technology (TSQ Quantiva mass spectrometer (MS); ThermoFisher Scientific). Identified peptide sequences and fragmentation ions thereof, so-called Transitions, for each peptide were found to be useful surrogates for monitoring marker proteins levels in a blood sample. Optimization was done on synthetic peptides which can be isotopically heavy labeled. Best peptides regarding signal to noise were selected. Optimal retention time and at least 4 best transitions were set up for each peptide.

### Exemplary MS Quantification and Choice of Peptides and Transitions

5uL of each clinical plasma sample was added to 20uL of 8M Urea/2.5% n-propanol/300mM Tris/10mM DTT pH 8.5 and incubated at 37C for one hour. 500mM iodoacetic acid prepared in 1M ammonium bicarbonate was added to each sample well and incubated in the dark at room temperature for one hour. 113uL of 50mM Tris/5mM CaCl2 pH 8.0 were added to each well. Trypsin (Thermo Fisher Scientific) was rehydrated with 150uL of 25mM acetic acid is added with a ratio 1:10 (total protein content:protease) and incubated at 37C for 20 hours. Digestion was finally quenched with the additional of 2uL of formic acid. Glucagon (1ng/uL) and standard heavy peptides were then added before injection.

SRM assays were developed on a triple quadrupole mass spectrometer TSQ Quantiva coupled with HPLC Ultimate 3000 (Thermo Fisher Scientific). Reverse phase separations were carried out in a 20 min linear gradient from 5 to 40% B, with a total run time of 40 min (Solvent A : Water 0.2% FA, Solvent B : ACN 0.2% FA). The flow rate during the linear gradient was set to 240 µL/min. The total injection volume was 160 µL for all samples and points on the curve. A 150 mm ×2.1 mm Accucore aQ column (ThermoFisher Scientific) was run at a temperature of 50°C.

Optimization was performed on heavy labeled synthetic peptides, incorporating 13C- and 15N-labeled arginine or lysine (ThermoFisher Scientific or New England Peptide). Individual instrument parameters such as collision energy, tube lens, and dwell time were automatically tested for every transition. After multiple iterations, the optimized list of peptides and transitions (i.e. highest intensity signal and least overlap with other transitions), and corresponding retention times were finalized with at least four fragment transitions per peptide chosen. Peptides were identified by co-eluting light and heavy-labeled transitions in the chromatographic separation. Pinpoint (Thermo Fisher Scientific) and Skyline (MacCoss Lab) softwares were used for time alignment, relative quantification of the transitions and targeted protein quantification.

**Relative and absolute quantifications of the markers were performed by employing the exemplary methods as described in the following.**

### Relative quantification:

1. Determining increased or decreased presence of the marker by comparing the SRM signature peak area from a given peptide detected in biological sample to the same SRM signature peak area of the same fragment peptide in at least a second, third, fourth or more biological samples.
2. Determining increased or decreased presence of the marker by comparing the SRM signature peak area from a given peptide detected in a biological sample to SRM signature peak areas developed from fragment peptides from other proteins, in other samples derived from different and separate biological sources, where the SRM signature peak area comparison between the 2 samples for a peptide fragment are normalized to amount of protein analyzed in each sample.
3. Determining increased or decreased presence of the marker by comparing the SRM signature peak area for a given peptide to the SRM signature peak areas from other fragment peptides derived from different proteins within the same biological sample in order to normalize changing levels of the maker to levels of other proteins that do not change their levels of expression under various cellular conditions.
4. These assays were applied to both unmodified fragment peptides and for modified fragment peptides, e.g. the histones protein, where the modifications included, but were not limited to phosphorylation and/or glycosylation, acetylation, methylation (mono, di, tri), citrullination, ubiquitinization and where the relative levels of modified peptides were determined in the same manner as determining relative amounts of unmodified peptides.

### Absolute quantification of a given peptide:

Comparing the SRM/MRM signature peak area for a given fragment peptide from the marker in an individual biological sample to the SRM/MRM signature peak area of an internal fragment peptide standard spiked into the protein lysate from the biological sample.

The internal standard was a labeled synthetic version of the fragment peptide from the marker protein that was being interrogated or the labeled recombinant protein. This standard was spiked into a sample in known amounts before or after digestion, and the SRM/MRM signature peak area was determined for both the internal fragment peptide standard and the native fragment peptide in the biological sample separately, followed by comparison of both peak areas.

Such an assay was applied to unmodified fragment peptides and modified fragment peptides, where the modifications included but are not limited to phosphorylation and/or glycosylation, acetylation, methylation (mono, di, tri), citrullination, ubiquitinization, and where the absolute levels of modified peptides were determined in the same manner as determining absolute levels of unmodified peptides.

Levels of histone H4 were also measured by immunoassay methods. Accordingly, the Histone H4 Immuno-Assay (H4 IA) consist of a mouse monoclonal antibody (mAb) raised against a synthetic peptide (amino acids 46-56 of SEQ ID NO: 1) coupled to MagPlex-C Micropheres (Luminex, Austin Texas), and a biotinylated sheep polyclonal antibody (pAb) raised against a synthetic peptide (amino acids 67-78 of SEQ ID NO 1). A synthetic peptide (amino acids 46-102 of SEQ ID NO: 1) was used as standard material. Samples were measured on a MAgPix with xPonent 4.2 System (Luminex, Austin Texas). Data was analyzed using 5 parameter logistic regression from JMP-12(SAS statistical software, UK).

Serum lactate levels were measured by colorimetric assay using the e501 module analyzer from Roche Diagnostics (Meylan, France). Reference limit for lactate was 0.5 - 2.2 mmol/L.

### Statistical analysis

All analyses were performed using the software R 3.0.2.The data is expressed as median and interquartile range [IQR] in brackets.As all analyzed biomarkers show highly right-skewed distributions, values were log₁₀-transformed prior to inclusion into regression models in order to decrease the impact of extreme values on the model fit.

Values below limit of quantification (LoQ) were replaced by a small value below LoQ. Missing values were not replaced. Each model includes all patients with complete data on all variables in the model. P-values <0.05 were considered as significant.Boxes in boxplots show the lower and upper quartile and the median (bold line). The whiskers extend to the most extreme data point which is no more than 1.5 times the interquartile range (IQR) from the box. All data points beyond the hinges are displayed as outliers. Between-group comparisons are performed using ANOVA on log₁₀-transformed biomarker values (clinical scores are not transformed). Post-hoc comparisons are analyzed using Tukey's honest significance distances. For dichotomous outcome variables, logistic regression models are used. Displayed results are the nested Likelihood-Ratio-χ² test (L.R. χ² and p-value) and the C index (Harrel).

Exemplary cut-offs are optimized via maximization of Youden's index, which is defined as 'Sensitivity + Specificity - 1'. The cut-off optimization was performed using the R library 'OptimalCutpoints'.

### RESULTS

The study population comprised 237 patients. Two patients (one patient without SIRS, one sepsis patient) had to be excluded from analyses due to conflicting documentation of mortality data. One hundred and seventy-two patients (73 %) presented with severe sepsis or septic shock, 15 patients (6 %) with SIRS and 49 patients (21 %) without SIRS. Median age was 67 [59-77 years] years. The majority of patients were male (60 %). Most frequent underlying conditions were cardiovascular diseases (35 %), diabetes mellitus (31 %) and malignancies (27 %) followed by respiratory disease (16 %), liver disease (12 %), renal disease (12 %) and immunodepression (7 %). Most frequent site of infection was the lower respiratory tract (46 %) and urinary tract (45 %). The SAPS II score (e.g. median 56 [40-69] points) and SOFA score (e.g. median 9 [6-12] points) were increased on admission. The observed organ failures were most frequently respiratory failure (61 %), circulatory shock (56 %) and renal failure (41 %). Accordingly, many patients required mechanical ventilation (MV) (78 %), vasopressors (68 %) and renal replacement therapy (RRT) (37 %) during ICU stay. All cause ICU mortality was 32 %, median length of ICU stay was 5.4 [2.5-10.6 days] days.

The following analysis is limited to patients with organ failures, hence to the 172 patients with severe sepsis and septic shock as described above. Patients are grouped according to their number of acute organ failures (1, 2, 3, 4, 5 or 6/7) in all Figures and Tables.

Different selected biomarkers and clinical scores correlate differently with the increasing number of organ failures in a critical ill patient. The levels of the histones increased significantly in subjects demonstrating four or more organ failures (Figure 1A to 1D). Accordingly, there is a correlation between the number of organ failures in a subject and the level of the histones. The histones H2A, H2B, H3 and H4, as well as aldolase B, are inferior to the other biomarkers tested in differentiating subjects having one, two or three organ failures compared (see below). Moreover, the level of MR-proADM (Figure 1E) or the level of copeptin increased with the rising number of organ failures (e.g. from 1 to 4 organ failures). Accordingly, there is a correlation between the number of organ failures in a subject and the level of MR-proADM. However, MR-proADM and copeptin are inferior in differentiating between subjects having multiple failures, e.g. 4, 5 or 6/7 organs compared to the histones (Figure 1E). For further biomarkers as lactate and the two clinical scores SOFA and SAPS II, their median level for each assigned organ failure group correlates positively with the whole range from 1 to 6/7 organ failures (Figure 1F).

When comparing groups of organ failures, PCT and copeptin are capable in differentiating patients with low amounts of organ failures with statistical significance, represented by a p-value <0.05, for example 1 organ failure versus 3 or 4 organ failures (Table 1).

MR-proADM, lactate, SAPS II and SOFA score have the broadest range and capability in distinguishing patients with different numbers of organ failures (Table 1). Histones H2A, H2B, H3 and H4 as well as aldolase B are inferior when comparing 1 or 2 organ failures with up to 5 organ failures. The histones significant value in dividing patients into the group of 3 versus 4 organ failures, where other biomarkers, such as copeptin, lactate, MR-proADM and PCT are inferior (Table 1).

**Table 1: Pairwise group comparison between the number of organ failures for selected biomarkers and clinical scores.**

P-values for each biomarker/score are given for the comparison of 1, 2 or 3 organ failure(s) (OF(s)) versus 2, 3, 4, 5 or 6/7 OFs, as indicated (post hoc analysis using Tukey's test). Statistically significant biomarkers/scores with p-values <0.05 are highlighted in grey for the according group comparison.

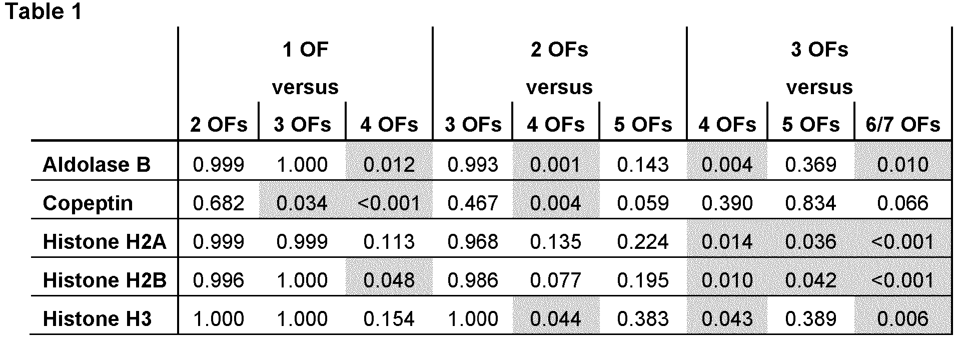

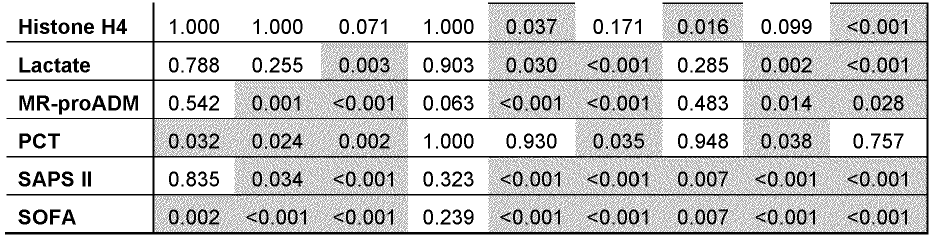

The predictive value of a biomarker/score to distinguish patients with up to 3 OFs from those with higher number of OFs was calculated by logistic regression models. Aside the clinical scores, MR-proADM, lactate and histone H2B showed the best predictive results (Table 2A).

In addition, the determination of a further marker (add-on marker) could further improve the prediction of organ failures in the subjects (Table 2B). For example, the SOFA score or the SAPS II score improved the prediction of the employed single markers, such as histones or MR-proADM. In addition, the combination of MR-proADM and the histones (H2A, H2B, H3 or H4) further improved the prediction of organ failures in comparison to the prediction of these markers used as a single marker, i.e. the determination of only, e.g. H2B or MR-proADM. The analysis also demonstrated that the best add-on-markers to the three best performing single markers/scores (SOFA, SAPS II, MR-proADM) include histones H2A, H2B, H4 and aldolase B (Table 2B).

The specificity for the prediction of a patient with ≥4 OFs is also highest for histones H2A, H2B and aldolase B (92.39 %, 88.04 %, 91.30 %, respectively), which is accompanied by a sensitivity of 45.00 %, 51.25 % and 45.00 %, respectively, as shown in Table 3 for the given cut-offs (reference levels). Such cut-off levels or reference levels were determined by mass spectrometry. In addition, exemplary reference levels of histone H4 were also determined by an immunoassay (IA) (Table 4). The specificity as well as the sensitivity of those levels determined using an immunoassay were comparable high as the reference levels determined by mass spectrometry. For example, a reference level of 35 ng/ml revealed a specificity of >80% and sensitivity of 53% (Table 4). Highest sensitivity is achieved by using the clinical scores SOFA and SAPS II or copeptin (87.50 %, 85.00 %, 78.21 %, respectively) with a corresponding specificity of 76.09 %, 72.83 % and 58.62 %, respectively (Table 3).

**Table 2: Logistic regression for outcome of ≥4 OFs for single biomarker/score models (2A) as well as combinations of two biomarkers/scores (2B).**

For each listed biomarker/score, the following model characteristics are indicated: the number of patients for the analysis, the number of patients with outcome ≥4 OFs, the likelihood ratio χ² as well as the C index. The list is sorted by the likelihood ratio, ranging from the highest to the lowest value. The p-value for all models in these tables is <0.001. For bivariate models (Table 2B) with a combination of two biomarkers/scores, all models with an add-on (increase of L.R. χ² >7) to the corresponding univariate model are shown. The table is limited to the three best single models from Table 2A (SOFA, SAPS II and MR-proADM). For each of these markers/scores, the three best add-on-models are highlighted in grey.

**Table 2A**

| | **N° of patients** | **N° of patients with N° of OFs ≥4** | **L.R. χ²** | **C index** |
|---|---|---|---|---|
| **SOFA** | 172 | 80 | 95.40 | 0.893 |
| **SAPS II** | 172 | 80 | 68.90 | 0.852 |
| **MR-proADM** | 172 | 80 | 46.00 | 0.780 |
| **Lactate** | 158 | 75 | 41.22 | 0.774 |
| **Histone H2B** | 172 | 80 | 29.00 | 0.713 |
| **Aldolase B** | 172 | 80 | 28.00 | 0.712 |
| **Histone H4** | 172 | 80 | 27.17 | 0.716 |
| **Copeptin** | 165 | 78 | 26.88 | 0.719 |
| **Histone H2A** | 172 | 80 | 26.70 | 0.710 |
| **Histone H3** | 172 | 80 | 19.90 | 0.689 |
| **Histone H4 (IA)** | 165 | 77 | 21,47 | 0.725 |
| **PCT** | 171 | 80 | 15.25 | 0.674 |

**Table 2B**

| | **N° of patients** | **N° of patients with N° of OFs ≥4** | **L.R. χ²** | **C index** |
|---|---|---|---|---|
| **SOFA + Histone H2B** | 172 | 80 | 110.92 | 0.914 |
| **SOFA + Histone H4** | 172 | 80 | 110.70 | 0.914 |
| **SOFA + Histone H2A** | 172 | 80 | 110.59 | 0.914 |
| **SOFA + Aldolase B** | 172 | 80 | 110.06 | 0.914 |
| **SOFA + Histone H3** | 172 | 80 | 105.19 | 0.906 |
| **SOFA + MR-proADM** | 172 | 80 | 104.04 | 0.906 |
| **SOFA + SAPS II** | 172 | 80 | 102.67 | 0.906 |
| **SOFA+Histone H4 (IA)** | 165 | 77 | 99.69 | 0.905 |
| **SAPS II + Histone H2A** | 172 | 80 | 88.93 | 0.881 |
| **SAPS II + Histone H4** | 172 | 80 | 88.88 | 0.882 |
| **SAPS II + Histone H2B** | 172 | 80 | 87.31 | 0.881 |
| **SAPS II** + **Aldolase B** | 172 | 80 | 85.71 | 0.876 |
| **SAPS II + Histone H3** | 172 | 80 | 84.35 | 0.875 |
| **SAPS II + MR-proADM** | 172 | 80 | 82.79 | 0.875 |
| **SAPS II + PCT** | 171 | 80 | 77.90 | 0.866 |
| **SAPS II + Lactate** | 158 | 75 | 75.94 | 0.874 |
| **MR-proADM + Aldolase B** | 172 | 80 | 68.50 | 0.834 |
| **MR-proADM + Histone H2B** | 172 | 80 | 60.02 | 0.820 |
| **MR-proADM + Histone H2A** | 172 | 80 | 58.92 | 0.817 |
| **MR-proADM + Histone H4** | 172 | 80 | 58.87 | 0.819 |
| **MR-proADM + Lactate** | 158 | 75 | 57.71 | 0.820 |
| **MR-proADM + Histone H3** | 172 | 80 | 55.57 | 0.809 |

**Table 3: Cut-offs for biomarkers and clinical scores for the prediction of ≥4 OFs.**

Biomarker levels/score values equal or above the given cut-off are determined to predict ≥4 OFs for a patient with the indicated sensitivity and specificity (PPV: positive predictive value; NPV: negative predictive value). The unit of the reference levels (cut-offs) is indicated. The reference levels determined by mass spectrometry are provided as arbitrary units (AU).

**Table 3**

| | **Cut-off** | **Sensitivity [%]** | **Specificity [%]** | **PPV [%]** | **NPV [%]** |
|---|---|---|---|---|---|
| **Aldolase B** | 42.1 AU | 45.00 | 91.30 | 81.82 | 65.63 |
| **Copeptin** | 79.71 pmol/L | 78.21 | 58.62 | 62.89 | 75.00 |
| **Histone H2A** | 42.4 AU | 45.00 | 92.39 | 83.72 | 65.89 |
| **Histone H2B** | 32.2 AU | 51.25 | 88.04 | 78.85 | 67.50 |
| **Histone H3** | 45.3 AU | 51.25 | 85.87 | 75.93 | 66.95 |
| **Lactate** | 3 mmol/L | 62.67 | 81.93 | 75.81 | 70.83 |
| **MR-proADM** | 5.97 nmol/L | 61.25 | 82.61 | 75.38 | 71.03 |
| **PCT** | 7.12 µg/L | 53.75 | 75.82 | 66.15 | 65.09 |
| **SAPS II** | 56 | 85.00 | 72.83 | 73.12 | 84.81 |
| **SOFA** | 10 | 87.50 | 76.09 | 76.09 | 87.50 |

Table 4: Cut-offs (reference levels) for Histone H4 for the prediction of ≥4 OFs.

Histone H4 levels/score values equal or above the given cut-off are determined to predict ≥4 OFs for a patient with the indicated sensitivity and specificity (PPV: positive predictive value; NPV: negative predictive value). Histone H4 levels were generated by measuring samples from patients using mass spectrometry (MS) or immunoassay (Luminex) techniques. The reference levels determined by MS were provided as arbitrary units (AU) and the reference levels determined by the immunoassay (Luminex or IA) are provided as ng/ml.

**Table 4:**

| | **cut off (<)** | **specificity.** | **sensitivity** |
|---|---|---|---|
| | AU | % | % |
| **MS** | 62 | >95% | 33,8% |
| | 41 | 93,0% | 45,0% |
| | 37 | >90% | 45,0% |
| | 28 | >85% | 48,8% |
| | 23 | 81,5% | 57,5% |
| | 22 | >80% | 57,5% |
| | 18 | >75% | 60,0% |
| | 16 | >70% | 62,5% |
| | 9 | 55,4% | 78,8% |

| | ng/mL | % | % |
|---|---|---|---|
| **Luminex** | 92 | >95% | 29,9% |
| | 43 | 92,2% | 52,0% |
| | 40 | >90% | 52,0% |
| | 35 | >85% | 53,3% |
| | 31 | >80% | 53,3% |
| | 25 | >75% | 57,1% |
| | 23 | 73,9% | 61,0% |
| | 19 | >70% | 64,9% |
| | 16 | 62,8% | 74,0% |
| | 11 | 51,6% | 79,2% |

References cited herein:
Albrich, W. C. and S. Harbarth (2015). Intensive Care Med 41(10): 1739-1751.
Bone, R. C., R. A. Balk, et al. (1992). Chest 101(6): 1644-1655.
Bouch, D. C. and J. P. Thompson (2008). Continuing Education in Anaesthesia, Critical Care & Pain 8(5): 181-185.
Breslow, M. J. and O. Badawi (2012). Chest 141(1): 245-252.
Ferreira, A. M. and Y. Sakr (2011). Semin Respir Crit Care Med 32(5): 543-551.
Knaus, W. A., E. A. Draper, et al. (1985). Crit Care Med 13(10): 818-829.
Le Gall, J. R., S. Lemeshow, et al. (1993). Jama 270(24): 2957-2963.
Mayr, V. D., M. W. Dunser, et al. (2006). Crit Care 10(6): R154.
Polderman, K. H., A. R. Girbes, et al. (2001). Anaesthesia 56(1): 47-50.
Vincent, J. L. (2006). Surg Infect (Larchmt) 7 Suppl 2: S69-72.
Vincent, J. L., A. de Mendonca, et al. (1998). of the European Society of Intensive Care
Medicine. Crit Care Med 26(11): 1793-1800.
Vincent, J. L. and R. Moreno (2010). Crit Care 14(2): 207.
Vincent, J. L., R. Moreno, et al. (1996). Intensive Care Med 22(7): 707-710.
Vincent, J. L., Y. Sakr, et al. (2006). Crit Care Med 34(2): 344-353.
Kutcher et al. (2012). Journal of Trauma and Acute Care Surgery 73(6): 1389-1394.
Ekaney et al. (2014). Critical Care 18(5): 1-9.
Abrams et al. (2013). American Journal of Respiratory and Critical Care Medicine 187(2): 160-169.
Kimura et al. (2006). Journal of Surgical Research 133(2): 102-112.
Potocki et al. (2012). Current Heart Failure Reports 9(3): 244-251.
Xu et al. (2009). Nature Medicine 15(11): 1318-1321.
Artunc et al. (2014). PLOS ONE 9(1): 1-8.
Meinders et al. (2012). European Heart Journal 33, Suppl. 1: 130.
Papathanassoglou et al. (2003). Biological Research for Nursing 5(2): 129-141.

## Claims

1. A method for the diagnosis and/or monitoring of the number of organ failures in a subject, wherein said subject is a critically ill patient, and wherein said method comprises
(i) determining a level of at least one histone in a blood, blood plasma, or blood serum sample of said subject; and
(ii) determining a level of proadrenomedullin (proADM) in a blood, blood plasma, or blood serum sample of said subject; wherein
(i1) said level of at least one histone is compared to a reference level of the at least one histone; and
(ii1) said level of proADM is compared to a reference level of proADM; and
(iii) wherein the number of organ failures in said subject is identified based on the comparison in step (i1) and (ii1).

2. The method of claim 1, wherein
(i) an increase in the level of at least one histone as compared to the reference level of at least one histone is indicative of the number of organ failures in said subject; and
(ii) an increase in the level of proADM as compared to the reference level proADM is indicative of the number of organ failures in said subject.

3. The method of claim 1 or 2,
(i) wherein the increase in the level of at least one histone as compared to the reference level of at least one histone is indicative of at least four organ failures in said subject; or
(ii) wherein the increase in the level of proADM of said subject as compared to said reference level of proADM is indicative of at least one organ failure.

4. The method of any one of the claims 1 to 3, wherein the reference level of at least one histone and/or the reference level of proADM is a level of at least one histone and/or proADM, as the case may be, from at least one reference subject, and wherein each of said at least one reference subject is healthy, or wherein said reference subject(s) has/have no organ organ failure.

5. The method of any one of claims 1 to 4, wherein proADM is midregional proadrenomedullin (MR-proADM) and wherein said at least one histone is histone H2B, histone H4, histone H2A and/or histone H3.

6. The method of any one of claims 1 to 5, wherein said reference level of at least one histone is about 10 ng/ml to about 100 ng/ml and/or said reference level of proADM is about 6 nmol/L.

7. The method of any one of claims 1 to 6, wherein said method further comprises determining at least one marker in said sample selected from the group consisting of a level of aldolase B, a level of copeptin, a level of lactate, a level of procalcitonin (PCT), a level of the heparin binding protein (HBP), and a level of soluble fms-like tyrosine kinase-1 (sFlt-1), and/or determining at least one parameter of said subject selected from the group consisting of the Acute Physiology and Chronic Health Evaluation II (APACHE II) score, the sequential organ failure assessment score (SOFA score), and the simplified acute physiology score (SAPSII).

8. The method of any one of claims 1 to 7, wherein said organ failure is at least one organ failure selected from the group consisting of circulatory shock, hematologic failure, liver failure, neurologic failure, renal failure, and respiratory failure.

9. The method of any one of claims 1 to 8, wherein said subject suffers from a disease or medical condition selected from the group consisting of cardiovascular disease, diabetes mellitus, malignancy, respiratory disease, liver disease, renal disease immunodepression, an inflammatory response related to infective and non-infective etiologies, systemic inflammatory response syndrome (SIRS), sepsis, severe sepsis, and septic shock.

10. The method of any one of claims 1 to 9, wherein said subject is admitted to an emergency department or wherein said subject is admitted to an intensive care unit.

11. The method of any one of claims 1 to 10, wherein said level of at least one histone and/or of proADM is/are determined using a method selected from the group consisting of mass spectrometry (MS), luminescence immunoassay (LIA), radioimmunoassay (RIA), chemiluminescence- and fluorescence- immunoassays, enzyme immunoassay (EIA), Enzyme-linked immunoassays (ELISA), luminescence-based bead arrays, magnetic beads based arrays, protein microarray assays, rapid test formats, and rare cryptate assay.

12. The method of any one of claims 1 to 11,
(i) wherein said at least one histone is histone H2B and wherein at least a peptide of the sequence spanning amino acid residues 41 to 69 of histone H2B according to SEQ ID NO: 4 is determined;
(ii) wherein said at least one histone is histone H4 and wherein at least a peptide of the sequence spanning amino acid residues 22 to 102 of histone H4 according to SEQ ID NO:1 is determined;
(iii) wherein said at least one histone is histone H3 and wherein at least a peptide of the sequence spanning amino acid residues 27 to 62 of histone H3 according to SEQ ID NO: 3 is determined; and/or
(iv) wherein said at least one histone is histone H2A and wherein at least a peptide of the sequence spanning amino acid residues 20 to 118 of histone H2A according to SEQ ID NO: 2 is determined.

13. A kit for carrying out the method according to any one of claims 1 to 12, wherein said kit comprises
(i) detection reagents for determining the level of at least one histone in said sample, comprising a ligand which specifically binds to said at least one histone, and reference data including the reference level of at least one histone, wherein an increase in the level of at least one histone in said sample as compared to the reference level of at least one histone is indicative of organ failure in said subject; and
(ii) detection reagents for determining the level of proADM in said sample, comprising a ligand which specifically binds to said proADM, and
reference data including the reference level of proADM, wherein an increase in the level of proADM in said sample as compared to the reference level of proADM is indicative of organ failure in said subject.

14. The use of the kit of claim 13 in the method of any one of the claims 1 to 12.

## Patentansprüche

1. Ein Verfahren zur Diagnose und/oder Überwachung der Anzahl von Organversagen bei einem Subjekt, wobei das Subjekt ein kritisch kranker Patient ist und wobei das Verfahren Folgendes umfasst
(i) Bestimmen des Spiegels von mindestens einem Histon in einer Blut-, Blutplasma- oder Blutserumprobe des Subjekts; und
(ii) Bestimmen des Proadrenomedullin-Spiegels (proADM) in einer Blut-, Blutplasma- oder Blutserumprobe des Subjekts; wobei
(i1) der Spiegel von mindestens einem Histon mit einem Referenzspiegel von mindestens einem Histon verglichen wird; und
(ii1) der proADM-Spiegel mit einem proADM-Referenzspiegel verglichen wird; und
(iii) wobei die Anzahl der Organversagen bei dem Subjekt auf der Grundlage des Vergleichs in Schritt (i1) und (ii1) ermittelt wird.

2. Verfahren nach Anspruch 1, wobei
(i) ein Anstieg des Spiegels von mindestens einem Histon im Vergleich zum Referenzspiegel von mindestens einem Histon ein Hinweis für die Anzahl der Organversagen bei dem Subjekt ist; und
(ii) ein Anstieg des proADM-Spiegels im Vergleich zum proADM-Referenzspiegel ein Hinweis für die Anzahl der Organversagen bei dem Subjekt ist.

3. Das Verfahren nach Anspruch 1 oder 2,
(i) wobei ein Anstieg des Spiegels von mindestens einem Histon im Vergleich zum Referenzspiegel von mindestens einem Histon ein Hinweis für mindestens vier Organversagen bei dem Subjekt ist; oder
(ii) wobei ein Anstieg des proADM-Spiegels des Subjekts im Vergleich zum proADM-Referenzspiegel ein Hinweis für mindestens ein Organversagen ist.

4. Das Verfahren nach einem der Ansprüche 1 bis 3, wobei der Referenzspiegel von mindestens einem Histon und/oder der proADM-Referenzspiegel ein Spiegel von mindestens einem Histon und/oder proADM ist, je nach Fall, von mindestens einem Referenzsubjekt, und wobei jeder der mindestens einen Referenzsubjekt(e) gesund ist, oder wobei das/die Referenzsubjekt(e) kein Organversagen erlitten hat/haben.

5. Das Verfahren nach einem der Ansprüche 1 bis 4, wobei proADM mittelregionales Proadrenomedullin (MR-proADM) ist und wobei das genannte Histon mindestens ein Histon H2B, Histon H4, Histon H2A und/oder Histon H3 ist.

6. Das Verfahren nach einem der Ansprüche 1 bis 5, wobei der Referenzspiegel von mindestens einem Histon etwa 10 ng/ml bis etwa 100 ng/ml beträgt und/oder der proADM-Referenzspiegel etwa 6 nmol/l beträgt.

7. Das Verfahren nach einem der Ansprüche 1 bis 6, wobei das Verfahren ferner die Bestimmung mindestens eines Markers in der Probe umfasst, ausgewählt aus der Gruppe, bestehend aus:
einem Aldolase-B-Spiegel, einem Copeptin-Spiegel, einem Laktat-Spiegel, einem Procalcitonin-Spiegel (PCT), einem Spiegel des Heparin-bindenden Proteins (HBP), und einem Spiegel löslicher fms-ähnlicher Tyrosinkinase-1 (sFlt-1), und/oder
die Bestimmung von mindestens einem Parameter des genannten Subjekts, ausgewählt aus der Gruppe bestehend aus dem Acute Physiology and Chronic Health Evaluation II (APACHE II) Score, dem Sequential Organ Failure Assessment Score (SOFA-Score) und dem Simplified Acute Physiology Score (SAPSII).

8. Das Verfahren nach einem der Ansprüche 1 bis 7, wobei das Organversagen mindestens ein Organversagen ausgewählt aus der Gruppe bestehend aus Kreislaufschock, hämatologischem Versagen, Leberversagen, neurologischem Versagen, Nierenversagen und Atemversagen, ist.

9. Das Verfahren nach einem der Ansprüche 1 bis 8, wobei das Subjekt an einer Krankheit oder einem medizinischen Zustand leidet, der aus der Gruppe ausgewählt wird, die aus Herz-Kreislauf-Erkrankungen, Diabetes mellitus, bösartigen Tumorerkrankungen (Malignität), Atemwegserkrankungen, Lebererkrankungen, Nierenerkrankungen, Immunsuppression und Entzündungsreaktionen im Zusammenhang mit infektiösen und nicht-infektiösen Ursachen, dem systemischen Entzündungsreaktionssyndrom (SIRS), Sepsis, schwerer Sepsis und septischem Schock bestehen.

10. Das Verfahren nach einem der Ansprüche 1 bis 9, wobei das Subjekt in einer Notaufnahme oder einer Intensivstation aufgenommen wird.

11. Das Verfahren nach einem der Ansprüche 1 bis 10, wobei der genannte Spiegel von mindestens einem Histon und/oder der proADM-Spiegel unter Verwendung eines Verfahrens bestimmt wird/werden, das aus der Gruppe ausgewählt ist, die aus Massenspektrometrie (MS), Lumineszenzimmunoassay (LIA), Radioimmunoassay (RIA), Chemilumineszenz- und Fluoreszenzimmunoassays, Enzymimmunoassay (EIA), Enzymgekoppelte Immunoassays (ELISA), Lumineszenz-basierte Bead-Arrays, Magnetbeadbasierte Arrays, Protein-Mikroarray-Assays, Schnelltestformate und seltenen Kryptat-Assays besteht.

12. Das Verfahren nach einem der Ansprüche 1 bis 11,
(i) wobei mindestens ein Histon ein Histon H2B ist und wobei mindestens ein Peptid der Sequenz, die die Aminosäurereste 41 bis 69 von Histon H2B gemäß SEQ ID NR: 4 umspannt, bestimmt wird;
(ii) wobei mindestens ein Histon ein Histon H4 ist und wobei mindestens ein Peptid der Sequenz, die die Aminosäurereste 22 bis 102 von Histon H4 gemäß SEQ ID NR:1 umspannt, bestimmt wird;
(iii) wobei mindestens ein Histon ein Histon H3 ist und wobei mindestens ein Peptid der Sequenz, welche die Aminosäurereste 27 bis 62 von Histon H3 gemäß SEQ ID NR:3 umspannt, bestimmt wird; und/oder
(iv) wobei mindestens ein Histon ein Histon H2A ist und wobei mindestens ein Peptid der Sequenz, die die Aminosäurereste 20 bis 118 von Histon H2A gemäß SEQ ID NR:2 umspannt, bestimmt wird.

13. Kit zur Durchführung des Verfahrens nach einem der Ansprüche 1 bis 12, wobei das Kit Folgendes umfasst
(i) Nachweisreagenzien zur Bestimmung des Spiegels von mindestens einem Histon in der Probe, die einen Liganden umfassen, der spezifisch an mindestens ein Histon bindet, und Referenzdaten, einschließlich des Referenzspiegels von mindestens einem Histon, wobei eine Erhöhung des Spiegels von mindestens einem Histon in der Probe im Vergleich zum Referenzspiegel von mindestens einem Histon ein Hinweis für ein Organversagen bei dem Subjekt ist; und
(ii) Nachweisreagenzien zur Bestimmung des proADM-Spiegels in der Probe, die einen Liganden umfassen, der spezifisch an das proADM bindet, und Referenzdaten, einschließlich des proADM-Referenzspiegels, wobei ein Anstieg des proADM-Spiegels in der Probe im Vergleich zum proADM-Referenzspiegel ein Hinweis für ein Organversagen bei dem Subjekt ist.

14. Verwendung des Kits von Anspruch 13 in dem Verfahren nach einem der Ansprüche 1 bis 12.

## Revendications

1. Procédé diagnostique et/ou de surveillance du nombre de défaillances d'organes chez un sujet, où ledit patient est un patient gravement malade, et où ledit procédé comprend
(i) la détermination d'un niveau d'au moins une histone dans un échantillon de sang, de plasma sanguin ou de sérum sanguin du patient concerné ; et
(ii) la détermination d'un niveau de pro-adrénomédulline (proADM) dans un échantillon de sang, de plasma sanguin ou de sérum sanguin du patient concerné ; où
(i1) le niveau d'au moins une histone est comparé à un niveau de référence d'au moins une histone ; et
(ii1) le niveau de proADM est comparé à un niveau de référence de proADM ; et
(iii) où le nombre de défaillances d'organes chez ledit sujet est identifié sur la base de la comparaison des étapes (i1) et (ii1).

2. Procédé selon la revendication 1, dans lequel
(i) une augmentation du niveau d'au moins une histone par rapport au niveau de référence d'au moins une histone est indicatif du nombre de défaillances d'organes chez ledit sujet ; et
(ii) une augmentation du niveau de proADM en comparaison avec le niveau de référence de proADM est indicatif du nombre de défaillances d'organes chez ledit sujet.

3. Procédé selon la revendication 1 ou 2,
(i) dans lequel l'augmentation du niveau d'au moins une histone par comparaison avec le niveau de référence d'au moins une histone est indicative d'au moins quatre défaillances d'organes chez ledit sujet ; ou
(ii) dans lequel l'augmentation du niveau de proADM dudit sujet par comparaison avec le niveau de référence de proADM est indicative d'au moins une défaillance d'organe.

4. Procédé selon l'une des revendications 1 à 3, dans lequel le niveau de référence d'au moins une histone et/ou le niveau de référence de proADM est un niveau d'au moins une histone et/ou de proADM, selon le cas, issu d'au moins un sujet de référence, et dans lequel au moins un sujet de référence est en bonne santé, ou dans lequel le(s) sujet(s) de référence ne présente(nt) aucune défaillance d'organe.

5. Procédé selon une des revendications 1 à 4, dans lequel la proADM est la pro-adrénomédulline mi-régionale (MR-proADM) et dans lequel au moins une histone est l'histone H2B, l'histone H4, l'histone H2A et/ou l'histone H3.

6. Procédé selon l'une des revendications 1 à 5, dans lequel ledit niveau de référence d'au moins une histone est d'environ 10 ng/ml à environ 100 ng/ml et/ou ledit niveau de référence de proADM est d'environ 6 nmol/l.

7. Procédé selon l'une des revendications 1 à 6, dans lequel ledit procédé comprend en outre la détermination d'au moins un marqueur dans ledit échantillon choisi dans le groupe composé d'un niveau d'aldolase B, d'un niveau de copeptine, d'un niveau de lactate, d'un niveau de procalcitonine (PCT), un niveau de protéine de liaison à l'héparine (HBP) et d'un niveau de tyrosine kinase-1 soluble de type fms (sFlt-1), et/ou la détermination d'au moins un paramètre dudit sujet choisi dans le groupe composé du score d'évaluation de physiologie aiguë et de santé chronique II (APACHE II), du score d'évaluation séquentielle des défaillances d'organe (score SOFA) et du score de physiologie aiguë simplifié (SAPSII).

8. Procédé selon l'une des revendications 1 à 7, dans lequel ladite défaillance d'organe est au moins une défaillance d'organe choisie dans le groupe comprenant choc circulatoire, insuffisance hématologique, insuffisance hépatique, insuffisance neurologique, insuffisance rénale et insuffisance respiratoire.

9. Procédé selon l'une des revendications 1 à 8, dans lequel ledit sujet est atteint d'une maladie ou d'une affection médicale choisie dans le groupe comprenant maladie cardiovasculaire, diabète de type 2, tumeur maligne, maladie respiratoire, maladie du foie, immunodépression liée à une maladie rénale, réponse inflammatoire liée à des étiologies infectieuses et non infectieuses, syndrome de réponse inflammatoire systémique (SRIS), sepsis, sepsis sévère et choc septique.

10. Procédé selon l'une des revendications 1 à 9, dans lequel ledit sujet est admis dans un service d'urgence ou dans lequel ledit sujet est admis dans une unité de soins intensifs.

11. Procédé selon l'une des revendications 1 à 10, dans lequel ledit niveau d'au moins un histone et/ou de proADM est ou sont déterminés au moyen d'un procédé choisi dans le groupe composé spectrométrie de masse (MS), immunodosage par luminescence (LIA), dosage radio-immunologique (RIA), immunodosage par chimiluminescence et fluorescence, immunodosage enzymatique (EIA), immunodosages liés aux enzymes (ELISA), réseaux de billes basés sur la luminescence, réseaux basés sur des billes magnétiques, dosages de microréseaux de protéines, formats de tests rapides et dosage de cryptates rares.

12. Le procédé selon l'une des revendications 1 à 11,
(i) dans lequel au moins une des histones est l'histone H2B et dans lequel au moins un peptide de la séquence couvrant les résidus d'acides aminés 41 à 69 de l'histone H2B selon SEQ ID No:4 est déterminé ;
(ii) dans lequel au moins une des histones est l'histone H4 et dans lequel au moins un peptide de la séquence couvrant les résidus d'acide aminé 22 à 102 de l'histone H4 selon SEQ ID NO:1 est déterminé ;
(iii) dans lequel au moins une des histones est l'histone H3 et dans lequel au moins un peptide de la séquence couvrant les résidus d'acides aminés 27 à 62 de l'histone H3 selon SEQ ID NO: 3 est déterminé ; et/ou
(iv) dans lequel au moins une des histones est l'histone H2A et dans lequel au moins un peptide de la séquence couvrant les résidus d'acides aminés 20 à 118 de l'histone H2A selon SEQ ID NO: 2 est déterminé.

13. Kit pour la mise en œuvre du procédé selon une des revendications 1 à 12, dans lequel ledit kit comprend
(i) des réactifs de détection pour déterminer le niveau d'au moins une histone dans ledit échantillon, comprenant un ligand qui se lie spécifiquement à ladite au moins une histone, et des données de référence comprenant le niveau de référence d'au moins une histone, dans lesquelles une augmentation du niveau d'au moins une histone dans ledit échantillon par comparaison avec le niveau de référence d'au moins une histone est indicatif d'une défaillance d'organe chez ledit sujet ; et
(ii) des réactifs de détection pour déterminer le niveau de proADM dans ledit échantillon, comprenant un ligand qui se lie spécifiquement à ladite proADM, et des données de référence comprenant le niveau de référence de proADM, dans lesquelles une augmentation du niveau de proADM dans ledit échantillon par rapport au niveau de référence de proADM est indicatif d'une défaillance d'organe chez ledit sujet.

14. L'utilisation du kit de la revendication 13 dans le procédé de l'une des revendications 1 à 12.
